(19) Europäisches Patentamt
European Patent Office
Office européen des brevets

(11) EP 2 305 787 A2

(12) EUROPEAN PATENT APPLICATION

(43) Date of publication:
**06.04.2011 Bulletin 2011/14**

(51) Int Cl.:
***C11D 3/50*** (2006.01) ***C11D 17/00*** (2006.01)

(21) Application number: **10189076.2**

(22) Date of filing: **15.02.2007**

(84) Designated Contracting States:
**AT BE BG CH CY CZ DE DK EE ES FI FR GB GR HU IE IS IT LI LT LU LV MC NL PL PT RO SE SI SK TR**
Designated Extension States:
**AL BA HR MK RS**

(30) Priority: **28.02.2006 US 777629 P**
**01.08.2006 US 834582 P**

(62) Document number(s) of the earlier application(s) in accordance with Art. 76 EPC:
**07705899.8 / 1 989 283**

(71) Applicant: **The Procter & Gamble Company**
**Cincinnati, OH 45202 (US)**

(72) Inventors:
- **Dihora, Jiten, Odhavji**
**Hamilton, OH 45011 (US)**
- **Sands, Peggy, Dorothy**
**Appleton, WI 54915 (US)**
- **Fossum, Renae, Dianna**
**Middletown, OH 45044 (US)**
- **York, David, William**
**Newcastle upon Tyne, Tyne and Wear NE209TB (GB)**
- **Lang, Matthew, Henry**
**Appleton, WI 54915 (US)**
- **Guinebretiere, Sandra Jacqueline**
**Appleton, WI 54913 (US)**

(74) Representative: **Goodier, Claire-Louise**
**N.V.Procter & Gamble Services Company S.A.**
**Temselaan 100**
**1853 Strombeek-Bever (BE)**

Remarks:
This application was filed on 27-10-2010 as a divisional application to the application mentioned under INID code 62.

(54) **Compositions comprising benefit agent containing delivery particles**

(57) The present invention relates to consumer product compositions benefit agent containing delivery particles, and processes for making and using the aforementioned particles and compositions. When employed in compositions, for example, cleaning or fabric care compositions, such particles increase the efficiency of benefit agent delivery, there by allowing reduced amounts of benefit agents to be employed. In addition to allowing the amount of benefit agent to be reduced, such particles allow a broad range of benefit agents to be employed. A method of improving the stability of a microcapsule slurry is also disclosed.

EP 2 305 787 A2

## Description

CROSS-REFERENCES TO RELATED APPLICATIONS

This application claims priority under 35 U.S.C. § 119(e) to U.S. Provisional Application Serial No. 60/834,582 filed August 1, 2006, and U.S. Provisional Application Serial No. 60/777,629 filed February 28, 2006.

FIELD OF INVENTION

The present application relates to benefit agent containing delivery particles, compositions comprising such particles, and processes for making and using such particles and compositions.

BACKGROUND OF THE INVENTION

Benefit agents, such as perfumes, silicones, waxes, flavors, vitamins and fabric softening agents, are expensive and generally less effective when employed at high levels in personal care compositions, cleaning compositions, and fabric care compositions. As a result, there is a desire to maximize the effectiveness of such benefit agents. One method of achieving such objective is to improve the delivery efficiencies of such benefit agents. Unfortunately, it is difficult to improve the delivery efficiencies of benefit agents as such agents may be lost do to the agents' physical or chemical characteristics, or such agents may be incompatible with other compositional components or the situs that is treated.

Accordingly, there is a need for a benefit agent containing delivery particle that provides improved benefit agent delivery efficiency.

SUMMARY OF THE INVENTION

The present invention relates to benefit agent containing delivery particles comprising a core material and a wall material that at least partially surrounds the core material. The present invention also relates to compositions comprising said particles, and processes for making and using such particles and compositions.

DETAILED DESCRIPTION OF THE INVENTION

Definitions

As used herein "consumer product" means baby care, beauty care, fabric & home care, family care, feminine care, health care, snack and/or beverage products or devices intended to be used or consumed in the form in which it is sold, and not intended for subsequent commercial manufacture or modification. Such products include but are not limited to diapers, bibs, wipes; products for and/or methods relating to treating hair (human, dog, and/or cat), including, bleaching, coloring, dyeing, conditioning, shampooing, styling; deodorants and antiperspirants; personal cleansing; cosmetics; skin care including application of creams, lotions, and other topically applied products for consumer use; and shaving products, products for and/or methods relating to treating fabrics, hard surfaces and any other surfaces in the area of fabric and home care, including: air care, car care, dishwashing, fabric conditioning (including softening), laundry detergency, laundry and rinse additive and/or care, hard surface cleaning and/or treatment, and other cleaning for consumer or institutional use; products and/or methods relating to bath tissue, facial tissue, paper handkerchiefs, and/or paper towels; tampons, feminine napkins; products and/or methods relating to oral care including toothpastes, tooth gels, tooth rinses, denture adhesives, tooth whitening; over-the-counter health care including cough and cold remedies, pain relievers, RX pharmaceuticals, pet health and nutrition, and water purification; processed food products intended primarily for consumption between customary meals or as a meal accompaniment (non-limiting examples include potato chips, tortilla chips, popcorn, pretzels, corn chips, cereal bars, vegetable chips or crisps, snack mixes, party mixes, multigrain chips, snack crackers, cheese snacks, pork rinds, corn snacks, pellet snacks, extruded snacks and bagel chips); and coffee.

As used herein, the term "cleaning composition" includes, unless otherwise indicated, granular or powder-form all-purpose or "heavy-duty" washing agents, especially cleaning detergents; liquid, gel or paste-form all-purpose washing agents, especially the so-called heavy-duty liquid types; liquid fine-fabric detergents; hand dishwashing agents or light duty dishwashing agents, especially those of the high-foaming type; machine dishwashing agents, including the various tablet, granular, liquid and rinse-aid types for household and institutional use; liquid cleaning and disinfecting agents, including antibacterial hand-wash types, cleaning bars, mouthwashes, denture cleaners, dentifrice, car or carpet shampoos, bathroom cleaners; hair shampoos and hair-rinses; shower gels and foam baths and metal cleaners; as well as cleaning auxiliaries such as bleach additives and "stain-stick" or pre-treat types, substrate-laden products such as dryer

added sheets, dry and wetted wipes and pads, nonwoven substrates, and sponges; as well as sprays and mists.

As used herein, the term "fabric care composition" includes, unless otherwise indicated, fabric softening compositions, fabric enhancing compositions, fabric freshening compositions and combinations there of.

As used herein, the phrase "benefit agent containing delivery particle" encompasses a benefit agent or core material and a wall material that at least partially surrounds the benefit agent or core material; encompasses microcapsules with a benefit agent or core material; encompasses microcapsules including perfume microcapsules; encompasses matrix materials such as a benefit agent surrounded at least partially by a solid or gelled carrier; encompasses matrix materials such as a benefit agent at least partially surrounded by a wall or wall-like network; encompasses aggregates of two materials where one material at least partially surrounds the other.

As used herein, the term "particle" is synonymous with the phrase "benefit agent containing delivery particle".

As used herein, the articles "a" and "an" when used in a claim, are understood to mean one or more of what is claimed or described.

As used herein, the terms "include", "includes" and "including" are meant to be non-limiting.

The test methods disclosed in the Test Methods Section of the present application should be used to determine the respective values of the parameters of Applicants' inventions.

Unless otherwise noted, all component or composition levels are in reference to the active portion of that component or composition, and are exclusive of impurities, for example, residual solvents or by-products, which may be present in commercially available sources of such components or compositions.

All percentages and ratios are calculated by weight unless otherwise indicated. All percentages and ratios are calculated based on the total composition unless otherwise indicated.

It should be understood that every maximum numerical limitation given throughout this specification includes every lower numerical limitation, as if such lower numerical limitations were expressly written herein. Every minimum numerical limitation given throughout this specification will include every higher numerical limitation, as if such higher numerical limitations were expressly written herein. Every numerical range given throughout this specification will include every narrower numerical range that falls within such broader numerical range, as if such narrower numerical ranges were all expressly written herein.

All documents cited are, in relevant part, incorporated herein by reference; the citation of any document is not to be construed as an admission that it is prior art with respect to the present invention.

## Benefit Agent Containing Delivery Particle

Applicants discovered that the problem of achieving effective and efficient benefit agent delivery can be solved in an economical manner when a benefit agent containing delivery particle having a certain combination of physical and chemical characteristics is employed. Such physical and chemical characteristics are defined by the following parameters: particle size coefficient of variation, fracture strength, benefit agent retention ratio and average particle size. Such parameters may be combined to yield a Delivery Index.

In one aspect, Applicants' particle comprises a core material and a wall material that at least partially surrounds the core material, said particle having a Delivery Index of at least about 0.05, at least about 7, or at least about 70.

In one aspect, Applicants' particle comprises a core material and a wall material that at least partially surrounds the core material, said particle having:

a.) a particle size coefficient of variation of from about 1.5 to about 6.0, from about 2.0 to about 3.5, or even from about 2.5 to about 3.2;

b.) a fracture strength of from about 0.1 psia to about 110 psia, from about 1 to about 50 psia, or even from about 4 to about 16 psia;

c.) a benefit agent retention ratio of from about 2 to about 110, from about 30 to about 90, or even from about 40 to about 70; and

d.) an average particle size of from about 1 micron to about 100 microns, from about 5 microns to about 80 microns, or even from about 15 microns to about 50 microns.

In one aspect of Applicants' invention, said particle may have and/or comprise any combination of the parameters described in the present specification.

Useful wall materials include materials selected from the group consisting of polyethylenes, polyamides, polystyrenes, polyisoprenes, polycarbonates, polyesters, polyacrylates, polyureas, polyurethanes, polyolefins, polysaccharides, epoxy resins, vinyl polymers, and mixtures thereof. In one aspect, useful wall materials include materials that are sufficiently impervious to the core material and the materials in the environment in which the benefit agent containing delivery particle will be employed, to permit the delivery benefit to be obtained. Suitable impervious wall materials include materials selected from the group consisting of reaction products of one or more amines with one or more aldehydes,

such as urea cross-linked with formaldehyde or gluteraldehyde, melamine cross-linked with formaldehyde; gelatin-polyphosphate coacervates optionally cross-linked with gluteraldehyde; gelatin-gum Arabic coacervates; cross-linked silicone fluids; polyamine reacted with polyisocyanates and mixtures thereof. In one aspect, the wall material comprises melamine cross-linked with formaldehyde.

Useful core materials include perfume raw materials, silicone oils, waxes, hydrocarbons, higher fatty acids, essential oils, lipids, skin coolants, vitamins, sunscreens, antioxidants, glycerine, catalysts, bleach particles, silicon dioxide particles, malodor reducing agents, dyes, brighteners, antibacterial actives, antiperspirant actives, cationic polymers and mixtures thereof. In one aspect, said perfume raw material is selected from the group consisting of alcohols, ketones, aldehydes, esters, ethers, nitriles alkenes. In one aspect the core material comprises a perfume. In one aspect, said perfume comprises perfume raw materials selected from the group consisting of alcohols, ketones, aldehydes, esters, ethers, nitriles alkenes and mixtures thereof. In one aspect, said perfume may comprise a perfume raw material selected from the group consisting of perfume raw materials having a boiling point (B.P.) lower than about 250˚C and a ClogP lower than about 3, perfume raw materials having a B.P. of greater than about 250˚C and a ClogP of greater than about 3, perfume raw materials having a B.P. of greater than about 250˚C and a ClogP lower than about 3, perfume raw materials having a B.P. lower than about 250˚C and a ClogP greater than about 3 and mixtures thereof. Perfume raw materials having a boiling point B.P. lower than about 250˚C and a ClogP lower than about 3 are known as Quadrant I perfume raw materials, perfume raw materials having a B.P. of greater than about 250˚C and a ClogP of greater than about 3 are known as Quadrant IV perfume raw materials, perfume raw materials having a B.P. of greater than about 250˚C and a ClogP lower than about 3 are known as Quadrant II perfume raw materials, perfume raw materials having a B.P. lower than about 250˚C and a ClogP greater than about 3 are known as a Quadrant III perfume raw materials. In one aspect, said perfume comprises a perfume raw material having B.P. of lower than about 250˚C. In one aspect, said perfume comprises a perfume raw material selected from the group consisting of Quadrant I, II, III perfume raw materials and mixtures thereof. In one aspect, said perfume comprises a Quadrant III perfume raw material. Suitable Quadrant I, II, III and IV perfume raw materials are disclosed in U.S. patent 6,869,923 B1.

In one aspect, said perfume comprises a Quadrant IV perfume raw material. While not being bound by theory, it is believed that such Quadrant IV perfume raw materials can improve perfume odor "balance". Said perfume may comprise, based on total perfume weight, less than about 30%, less than about 20%, or even less than about 15% of said Quadrant IV perfume raw material.

The perfume raw materials and accords may be obtained from one or more of the following companies Firmenich (Geneva, Switzerland), Givaudan (Argenteuil, France), IFF (Hazlet, NJ), Quest (Mount Olive, NJ), Bedoukian (Danbury, CT), Sigma Aldrich (St. Louis, MO), Millennium Specialty Chemicals (Olympia Fields, IL), Polarone International (Jersey City, NJ), Fragrance Resources (Keyport, NJ), and Aroma & Flavor Specialties (Danbury, CT).

<u>Process of making Benefit Agent Containing Delivery Particles</u>

The particle disclosed in the present application may be made via the teachings of USP 6,592,990 B2 and/or USP 6,544,926 B1 and the examples disclosed herein.

Anionic emulsifiers are typically used during the capsule making process to emulsify the benefit agent prior to microcapsule formation. While not being bound by theory, it is believed that the anionic materials adversely interact with the cationic surfactant actives that are often found in compositions such as fabric care compositions - this may yield an aesthetically unpleasing aggregation of particles that are employed in said composition. In addition to the unacceptable aesthetics, such aggregates may result in rapid phase separation of the particles from the bulk phase. Applicants discovered that such aggregates can be prevented by the addition of certain aggregate inhibiting materials including materials selected from the group consisting of salts, polymers and mixtures thereof. Useful aggregate inhibiting materials include, divalent salts such as magnesium salts, for example, magnesium chloride, magnesium acetate, magnesium phosphate, magnesium formate, magnesium boride, magnesium titanate, magnesium sulfate heptahydrate; calcium salts, for example, calcium chloride, calcium formate, calcium calcium acetate, calcium bromide; trivalent salts, such as aluminum salts, for example, aluminum sulfate, aluminum phosphate, aluminum chloride n-hydrate and polymers that have the ability to suspend anionic particles such as soil suspension polymers, for example, polyamines (polyethylene imines, alkoxylated polyethylene imines, polyquaternium-6 and polyquaternium-7.

In one aspect, Calcium Formate and/or formic acid may be added to an aqueous slurry of microcapsules, for example, perfume microcapsules. Calcium Formate and/or formic acid is typically combined with, based on total slurry weight, at a level of from about 0.6 wt% to about 3 wt.%, from about 1 wt% to about 2 wt. % or even from about 1.2 wt% to about 1.5 wt. %, said microcapsule slurry. Calcium Formate and/or formic acid may provide the following benefits: slurry phase separation inhibition, aggregate formation inhibition and microbial inhibition. Typically, the aforementioned microbial inhibition is achieved when the slurry and/or product comprising said slurry has a pH of 3.8 or less. Calcium Formate may be obtained from Perstorp Inc., of Toledo, Ohio U.S.A. and formic acid may be obtained from Aldrich, P.O. Box 2060, Milwaukee, WI 53201, USA.

In one aspect of the invention, benefit agent containing delivery particles are manufactured and are subsequently coated with a material to reduce the rate of leakage of the benefit agent from the particles when the particles are subjected to a bulk environment containing, for example, surfactants, polymers, and solvents. Non-limiting examples of coating materials that can serve as barrier materials include materials selected from the group consisting of polyvinyl pyrrolidone homopolymer, and its various copolymers with styrene, vinyl acetate, imidazole, primary and secondary amine containing monomers, methyl acrylate, polyvinyl acetal, maleic anhydride; polyvinyl alcohol homopolymer, and its various copolymers with vinyl acetate, 2-acrylamide-2-methylpropane sulfonate, primary and secondary amine containing monomers, imidazoles, methyl acrylate; polyacrylamides; polyacrylic acids; microcrystalline waxes; paraffin waxes; modified polysaccharides such as waxy maize or dent corn starch, octenyl succinated starches, derivatized starches such as hydroxyethylated or hydroxypropylated starches, carrageenan, guar gum, pectin, xanthan gum; modified celluloses such as hydrolyzed cellulose acetate, hydroxy propyl cellulose, methyl cellulose, and the like; modified proteins such as gelatin; hydrogenated and nonhydrogenated polyalkenes; fatty acids; hardened shells such as urea crosslinked with formaldehyde, gelatin-polyphosphate, melamine-formaldehyde, polyvinyl alcohol crosslinked with sodium tetraborate or gluteraldehyde; latexes of styrene-butadiene, ethyl cellulose, inorganic materials such as clays including magnesium silicates, aluminosilicates; sodium silicates, and the like; and mixtures thereof. Such materials can be obtained from CP Kelco Corp. of San Diego, California, USA; Degussa AG or Dusseldorf, Germany; BASF AG of Ludwigshafen, Germany; Rhodia Corp. of Cranbury, New Jersey, USA; Baker Hughes Corp. of Houston, Texas, USA; Hercules Corp. of Wilmington, Delaware, USA; Agrium Inc. of Calgary, Alberta, Canada, ISP of New Jeresy U.S.A.. In one aspect wherein the particle is employed in a fabric conditioning composition, the coating material comprises sodium silicate. While not being bound by theory, it is believed that sodium silicate's solubility at high pH, but poor solubility at low pH makes it an ideal material for use on particles that may be used in compositions that are formulated at pH below 7 but used in an environment wherein the pH is greater or equal to 7. The benefit agent containing delivery particles made be made by following the procedure described in USP 6,592,990. However, the coating aspect of the present invention is not limited to the benefit agent containing delivery particles of the present invention as any benefit agent containing delivery particle may benefit from the coatings and coating processes disclosed herein.

Suitable equipment for use in the processes disclosed herein may include continuous stirred tank reactors, homogenizers, turbine agitators, recirculating pumps, paddle mixers, ploughshear mixers, ribbon blenders, vertical axis granulators and drum mixers, both in batch and, where available, in continuous process configurations, spray dryers, and extruders. Such equipment can be obtained from Lodige GmbH (Paderborn, Germany), Littleford Day, Inc. (Florence, Kentucky, U.S.A.), Forberg AS (Larvik, Norway), Glatt Ingenieurtechnik GmbH (Weimar, Germany), Niro (Soeborg, Denmark), Hosokawa Bepex Corp. (Minneapolis, Minnesota, USA), Arde Barinco (New Jersey, USA).

Formaldehyde Scavenging

In one aspect, benefit agent containing delivery particles may be combined with a formaldehyde scavenger. In one aspect, such benefit agent containing delivery particles may comprise the benefit agent containing delivery particles of the present invention. Suitable formaldehyde scavengers include materials selected from the group consisting of sodium bisulfite, urea, ethylene urea, cysteine, cysteamine, lysine, glycine, serine, carnosine, histidine, glutathione, 3,4-diaminobenzoic acid, allantoin, glycouril, anthranilic acid, methyl anthranilate, methyl 4-aminobenzoate, ethyl acetoacetate, acetoacetamide, malonamide, ascorbic acid, 1,3-dihydroxyacetone dimer, biuret, oxamide, benzoguanamine, pyroglutamic acid, pyrogallol, methyl gallate, ethyl gallate, propyl gallate, triethanol amine, succinamide, thiabendazole, benzotriazol, triazole, indoline, sulfanilic acid, oxamide, sorbitol, glucose, cellulose, poly(vinyl alcohol), partially hydrolyzed poly(vinylformamide), poly(vinyl amine), poly(ethylene imine), poly(oxyalkyleneamine), poly(vinyl alcohol)-co-poly (vinyl amine), poly(4-aminostyrene), poly(l-lysine), chitosan, hexane diol, ethylenediamine-N,N'-bisacetoacetamide, N-(2-ethylhexyl)acetoacetamide, 2-benzoylacetoacetamide, N-(3-phenylpropyl)acetoacetamide, lilial, helional, melonal, triplal, 5,5-dimethyl-1,3-cyclohexanedione, 2,4-dimethyl-3-cyclohexenecarboxaldehyde, 2,2-dimethyl-1,3-dioxan-4,6-dione, 2-pentanone, dibutyl amine, triethylenetetramine, ammonium hydroxide, benzylamine, hydroxycitronellol, cyclohexanone, 2-butanone, pentane dione, dehydroacetic acid, or a mixture thereof. These formaldehyde scavengers may be obtained from Sigma/Aldrich/Fluka of St. Louis, MO. U.S.A. or PolySciences, Inc. of Warrington, PA U.S.A.

Such formaldehyde scavengers are typically combined with a slurry containing said benefit agent containing delivery particle, at a level, based on total slurry weight, of from about 2 wt.% to about 18 wt.%, from about 3.5 wt.% to about 14 wt.% or even from about 5 wt.% to about 13 wt.%.

In one aspect, such formaldehyde scavengers may be combined with a product containing a benefit agent containing delivery particle, said scavengers being combined with said product at a level, based on total product weight, of from about 0.005% to about 0.8%, alternatively from about 0.03% to about 0.5%, alternatively from about 0.065% to about 0.25% of the product formulation,.

In another aspect, such formaldehyde scavengers may be combined with a slurry containing said benefit agent containing delivery particle, at a level, based on total slurry weight, of from about 2 wt.% to about 14 wt.%, from about

3.5 wt.% to about 14 wt.% or even from about 5 wt.% to about 14 wt.% and said slurry may be added to a product matrix to which addition an identical or different scavenger may be added at a level, based on total product weight, of from about 0.005% to about 0.5%, alternatively from about 0.01% to about 0.25%, alternatively from about 0.05% to about 0.15% of the product formulation,

In one aspect, one or more of the aforementioned formaldehyde scavengers may be combined with a liquid fabric enhancing product containing a benefit agent containing delivery particle at a level, based on total liquid fabric enhancing product weight, of from 0.005% to about 0.8%, alternatively from about 0.03% to about 0.4%, alternatively from about 0.06% to about 0.25% of the product formulation

In one aspect, such formaldehyde scavengers may be combined with a liquid laundry detergent product containing a benefit agent containing delivery particle, said scavengers being selected from the group consisting of sodium bisulfite, urea, ethylene urea, cysteine, cysteamine, lysine, glycine, serine, carnosine, histidine, glutathione, 3,4-diaminobenzoic acid, allantoin, glycouril, anthranilic acid, methyl anthranilate, methyl 4-aminobenzoate, ethyl acetoacetate, acetoacetamide, malonamide, ascorbic acid, 1,3-dihydroxyacetone dimer, biuret, oxamide, benzoguanamine, pyroglutamic acid, pyrogallol, methyl gallate, ethyl gallate, propyl gallate, triethanol amine, succinamide, thiabendazole, benzotriazol, triazole, indoline, sulfanilic acid, oxamide, sorbitol, glucose, cellulose, poly(vinyl alcohol), partially hydrolyzed poly(vinylformamide), poly(vinyl amine), poly(ethylene imine), poly(oxyalkyleneamine), poly(vinyl alcohol)-co-poly (vinyl amine), poly(4-aminostyrene), poly(l-lysine), chitosan, hexane diol, ethylenediamine-N,N'-bisacetoacetamide, N-(2-ethylhexyl)acetoacetamide, 2-benzoylacetoacetamide, N-(3-phenylpropyl)acetoacetamide, lilial, helional, melonal, triplal, 5,5-dimethyl-1,3-cyclohexanedione, 2,4-dimethyl-3-cyclohexenecarboxaldehyde, 2,2-dimethyl-1,3-dioxan-4,6-dione, 2-pentanone, dibutyl amine, triethylenetetramine, ammonium hydroxide, benzylamine, hydroxycitronellol, cyclohexanone, 2-butanone, pentane dione, dehydroacetic acid and mixtures thereof, and combined with said liquid laundry detergent product at a level, based on total liquid laundry detergent product weight, of from about 0.003 wt.% to about 0.20 wt.%, from about 0.03 wt.% to about 0.20 wt.% or even from about 0.06 wt.% to about 0.14 wt.%.

In one aspect, such formaldehyde scavengers may be combined with a hair conditioning product containing a benefit agent containing delivery particle, at a level, based on total hair conditioning product weight, of from about 0.003 wt. % to about 0.30 wt.%, from about 0.03 wt.% to about 0.20 wt.% or even from about 0.06 wt.% to about 0.14 wt.%., said selection of scavengers being identical to the list of scavengers in the previous paragraph relating to a liquid laundry detergent product.

<u>Compositions Comprising Benefit Agent Containing Delivery Particles</u>

Applicants' compositions comprise an embodiment of the particle disclosed in the present application. In one aspect, said composition is a consumer product. While the precise level of particle that is employed depends on the type and end use of the composition, a composition may comprise from about 0.01 to about 10, from about 0.1 to about 10, or even from about 0.2 to about 5 weight % of said particle based on total composition weight. In one aspect, a cleaning composition may comprise, from about 0.1 to about 1 weight % of such particle based on total cleaning composition weight of such particle. In one aspect, a fabric treatment composition may comprise, based on total fabric treatment composition weight, form about 0.01 to about 10% of such particle.

Aspects of the invention include the use of the particles of the present invention in laundry detergent compositions (e.g., TIDE™), hard surface cleaners (e.g., MR CLEAN™), automatic dishwashing liquids (e.g., CASCADE™), dishwashing liquids (e.g., DAWN™), and floor cleaners (e.g., SWIFFER™). Non-limiting examples of cleaning compositions may include those described in U.S. Pat. Nos. 4,515,705; 4,537,706; 4,537,707; 4,550,862; 4,561,998; 4,597,898; 4,968,451; 5,565,145; 5,929,022; 6,294,514; and 6,376,445. The cleaning compositions disclosed herein are typically formulated such that, during use in aqueous cleaning operations, the wash water will have a pH of between about 6.5 and about 12, or between about 7.5 and 10.5. Liquid dishwashing product formulations typically have a pH between about 6.8 and about 9.0. Cleaning products are typically formulated to have a pH of from about 7 to about 12. Techniques for controlling pH at recommended usage levels include the use of buffers, alkalis, acids, etc., and are well known to those skilled in the art.

Fabric treatment compositions disclosed herein typically comprise a fabric softening active ("FSA"). Suitable fabric softening actives, include, but are not limited to, materials selected from the group consisting of quats, amines, fatty esters, sucrose esters, silicones, dispersible polyolefins, clays, polysaccharides, fatty oils, polymer latexes and mixtures thereof.

Suitable quats include but are not limited to, materials selected from the group consisting of ester quats, amide quats, imidazoline quats, alkyl quats, amdioester quats and mixtures thereof. Suitable ester quats include but are not limited to, materials selected from the group consisting of monoester quats, diester quats, triester quats and mixtures thereof. Suitable amide quats include but are not limited to, materials selected from the group consisting of monoamide quats, diamide quats and mixtures thereof. Suitable alkyl quats include but are not limited to, materials selected from the group consisting of mono alkyl quats, dialkyl quats quats, trialkyl quats, tetraalkyl quats and mixtures thereof.

Suitable amines include but are not limited to, materials selected from the group consisting of esteramines, amidoamines, imidazoline amines, alkyl amines, amdioester amines and mixtures thereof. Suitable ester amines include but are not limited to, materials selected from the group consisting of monoester amines, diester amines, triester amines and mixtures thereof. Suitable amido quats include but are not limited to, materials selected from the group consisting of monoamido amines, diamido amines and mixtures thereof. Suitable alkyl amines include but are not limited to, materials selected from the group consisting of mono alkylamines, dialkyl amines quats, trialkyl amines, and mixtures thereof.

In one embodiment, the FSA is a quaternary ammonium compound suitable for softening fabric in a rinse step. In one embodiment, the FSA is formed from a reaction product of a fatty acid and an aminoalcohol obtaining mixtures of mono-, di-, and, in one embodiment, triester compounds. In another embodiment, the FSA comprises one or more softener quaternary ammonium compounds such, but not limited to, as a monoalkyquaternary ammonium compound, dialkylquaternary ammonium compound, a diamido quaternary compound, a diester quaternary ammonium compound, or a combination thereof.

In one aspect, the FSA comprises a diester quaternary ammonium or protonated diester ammonium (hereinafter "DQA") compound composition. In certain embodiments of the present invention, the DQA compound compositions also encompass diamido FSAs and FSAs with mixed amido and ester linkages as well as the aforementioned diester linkages, all herein referred to as DQA.

A first type of DQA ("DQA (1)") suitable as a FSA in the present composition includes a compound comprising the formula:

$$\{R_{(4-m)} - N^{+} - [(CH_2)_n - Y - R^1]_m\} \; X^- \quad (1)$$

wherein each R substituent is either hydrogen, a short chain $C_1$-$C_6$, preferably $C_1$-$C_3$ alkyl or hydroxyalkyl group, e.g., methyl (most preferred), ethyl, propyl, hydroxyethyl, hydroxypropyl and the like, poly ($C_2$-$C_3$ alkoxy), preferably polyethoxy, benzyl, or mixtures thereof; each m is 2 or 3; each n is from 1 to about 4, preferably 2; each Y is -O-(O)C-, -C(O)-O-, -NR-C(O)-, or -C(O)-NR- and it is acceptable for each Y to be the same or different; the sum of carbons in each $R^1$, plus one when Y is -O-(O)C- or -NR-C(O) -, is $C_{12}$-$C_{22}$, preferably $C_{14}$-$C_{20}$, with each $R^1$ being a hydrocarbyl, or substituted hydrocarbyl group; it is acceptable for $R^1$ to be unsaturated or saturated and branched or linear and preferably it is linear; it is acceptable for each $R^1$ to be the same or different and preferably these are the same; and $X^-$ can be any softener-compatible anion, preferably, chloride, bromide, methylsulfate, ethylsulfate, sulfate, phosphate, and nitrate, more preferably chloride or methyl sulfate. Preferred DQA compounds are typically made by reacting alkanolamines such as MDEA (methyldiethanolamine) and TEA (triethanolamine) with fatty acids. Some materials that typically result from such reactions include N,N-di(acyl-oxyethyl)-N,N-dimethylammonium chloride or N,N-di(acyl-oxyethyl)-N,N-methylhydroxyethylammonium methylsulfate wherein the acyl group is derived from animal fats, unsaturated, and polyunsaturated, fatty acids, e.g., tallow, hardened tallow, oleic acid, and/or partially hydrogenated fatty acids, derived from vegetable oils and/or partially hydrogenated vegetable oils, such as, canola oil, safflower oil, peanut oil, sunflower oil, corn oil, soybean oil, tall oil, rice bran oil, palm oil, etc. Non-limiting examples of suitable fatty acids are listed in USP 5,759,990 at column 4, lines 45-66. In one embodiment the FSA comprises other actives in addition to DQA (1) or DQA. In yet another embodiment, the FSA comprises only DQA (1) or DQA and is free or essentially free of any other quaternary ammonium compounds or other actives. In yet another embodiment, the FSA comprises the precursor amine that is used to produce the DQA.

In another aspect of the invention, the FSA comprises a compound, identified as DTDMAC comprising the formula:

$$[R_{(4-m)} - N^{+} - R^1_m] \; A^-$$

wherein each m is 2 or 3, each $R^1$ is a $C_6$-$C_{22}$, preferably $C_{14}$-$C_{20}$, but no more than one being less than about $C_{12}$ and then the other is at least about $C_{16}$, hydrocarbyl, or substituted hydrocarbyl substituent, preferably $C_{10}$-$C_{20}$ alkyl or alkenyl (unsaturated alkyl, including polyunsaturated alkyl, also referred to sometimes as "alkylene"), most preferably $C_{12}$-$C_{18}$ alkyl or alkenyl, and branched or unbranched. In one embodiment; each R is H or a short chain $C_1$-$C_6$, preferably $C_1$-$C_3$ alkyl or hydroxyalkyl group, e.g., methyl (most preferred), ethyl, propyl, hydroxyethyl, and the like, benzyl, or $(R^2O)_{2-4}H$ where each $R^2$ is a $C_1$-$C_6$ alkylene group; and A- is a softener compatible anion, preferably, chloride, bromide, methylsulfate, ethylsulfate, sulfate, phosphate, or nitrate; more preferably chloride or methyl sulfate. Examples of these FSAs include dialkydimethylammonium salts and dialkylenedimethylammonium salts such as ditallowdimethylammonium chloride and ditallowdimethylammonium methylsulfate. Examples of commercially available dialkyl(ene)dimethylammonium salts usable in the present invention are dihydrogenated tallow dimethyl ammonium chloride and ditallowdimethyl ammonium chloride available from Degussa under the trade names Adogen® 442 and Adogen® 470 respectively. In one embodiment the FSA comprises other actives in addition to DTDMAC. In yet another embodiment, the FSA comprises only compounds of the DTDMAC and is free or essentially free of any other quaternary ammonium compounds or other actives.

In one embodiment, the FSA comprises an FSA described in U.S. Pat. Pub. No. 2004/0204337 A1, published Oct. 14, 2004 to Corona et al., from paragraphs 30 — 79.

In another embodiment, the FSA is one described in U.S. Pat. Pub. No. 2004/0229769 A1, published Nov. 18, 2005, to Smith et al., on paragraphs 26 - 31; or U.S. Pat. No. 6,494,920, at column 1, line 51 *et seq.* detailing an "esterquat" or a quaternized fatty acid triethanolamine ester salt.

In one embodiment, the FSA is chosen from at least one of the following: ditallowoyloxyethyl dimethyl ammonium chloride, dihydrogenated-tallowoyloxyethyl dimethyl ammonium chloride, ditallow dimethyl ammonium chloride, dihydrogenatedtallow dimethyl ammonium chloride, ditallowoyloxyethyl methylhydroxyethylammonium methyl sulfate, dihydrogenated-tallowoyloxyethyl methyl hydroxyethylammonium chloride, or combinations thereof.

Typical minimum levels of incorporation of the FSA in the present fabric care compositions are at least about 1 %, alternatively at least about 2%, alternatively at least about at least about 3%, alternatively at least about at least about 5%, alternatively at least about 10%, and alternatively at least about 12%, by weight of the fabric care composition. The fabric care composition may typically comprise maximum levels of FSA of about less than about 90%, alternatively less than about 40%, alternatively less than about 30%, alternatively less than about 20%, by weight of the composition.

Cationic Starch

One aspect of the invention provides a fabric softening composition comprising a cationic starch as a fabric softening active. In one embodiment, the fabric care compositions of the present invention generally comprise cationic starch at a level of from about 0.1 % to about 7%, alternatively from about 0.1% to about 5%, alternatively from about 0.3% to about 3%, and alternatively from about 0.5% to about 2.0%, by weight of the composition. Cationic starch as a fabric softening active is described in U.S. Pat. Pub. 2004/0204337 A1, published Oct. 14, 2004, to Corona et al., at paragraphs 16 - 32. Suitable cationic starches for use in the present compositions are commercially-available from Cerestar under the trade name C*BOND® and from National Starch and Chemical Company under the trade name CATO® 2A.

Silicone

In one embodiment, the fabric softening composition comprises a silicone. Suitable levels of silicone may comprise from about 0.1% to about 50%, alternatively from about 1% to about 40%, alternatively from about 2% to about 30%, alternatively from about 3% to about 20% by weight of the composition. Non limiting examples of silicones include those described in U.S. Pat. Pub. No. 2002/0077265 A1, to Buzzacarini et al., published June 20, 2002 at paragraphs 51 - 57. Useful silicones can be any silicone comprising compound. In one embodiment, the silicone is a polydialkylsilicone, alternatively a polydimethyl silicone (polydimethyl siloxane or "PDMS"), or a derivative thereof. In another embodiment, the silicone is chosen from an aminofunctional silicone, alkyloxylated silicone, ethoxylated silicone, propoxylated silicone, ethoxylated/propoxylated silicone, quaternary silicone, or combinations thereof. Other useful silicone materials may include materials of the formula:

$$HO[Si(CH_3)_2\text{-}O]_x\{Si(OH)[(CH_2)_3\text{-}NH\text{-}(CH_2)_2\text{-}NH_2]O\}_yH$$

wherein x and y are integers which depend on the molecular weight of the silicone, preferably has a molecular weight such that the silicone exhibits a viscosity of from about 500 cSt to about 500,000 cSt at 25° C. This material is also known as "amodimethicone".

In one embodiment, the silicone is one comprising a relatively high molecular weight. A suitable way to describe the molecular weight of a silicone includes describing its viscosity. A high molecular weight silicone is one having a viscosity of from about 1,000 cSt to about 3,000,000 cSt, preferably from about 6,000 cSt to about 1,000,000 cSt, alternatively about 7,000 cSt to about 1,000,000 cSt, alternatively 8,000 cSt to about 1,000,000 cSt, alternatively from about 10,000 cSt to about 600,000 cSt, alternatively from about 100,000 cSt to about 350,000 cSt. In yet another embodiment, the silicone is a PDMS or derivatives thereof, having a viscosity from about 60,000 cSt to about 600,000 cSt, alternatively from about 75,000 cSt to about 350,000 cSt, and alternatively at least about 100,000 cSt. In yet another embodiment, the viscosity of the aminofunctional silicone can be low (e.g., from about 50 cSt to about 100,000 cSt).

Other Fabric Softening Agents

In addition to or in lieu of fabric softening actives herein described, other materials can be used as fabric softening agents in compositions of the present invention. Non-limiting examples of these other agents include: clays, fatty oils, such as fatty acids, triglycerides, fatty alcohols, fatty esters, fatty amides, fatty amines; sucrose esters, dispersible polyethylenes, and polymer latexes. Examples of fatty acids are described in WO06007911A1 and WO06007899A1. Clays are described in U.S. Pat. Pub. No. 2004/0142841 A1 published Jul. 22, 2004, to de Buzzaccarini et al., from paragraphs 74 - 99.

Nonionic fabric care benefit agents can comprise sucrose esters, and are typically derived from sucrose and fatty acids. Sucrose ester is composed of a sucrose moiety having one or more of its hydroxyl groups esterified.

Sucrose is a disaccharide having the following formula:

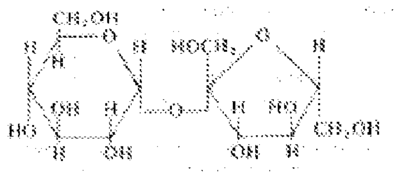

Alternatively, the sucrose molecule can be represented by the formula: $M(OH)_8$ , wherein M is the disaccharide backbone and there are total of 8 hydroxyl groups in the molecule.

Thus, sucrose esters can be represented by the following formula:

$$M(OH)_{8-x}(OC(O)R^1)_x$$

wherein x is the number of hydroxyl groups that are esterified, whereas (8-x) is the hydroxyl groups that remain unchanged; x is an integer selected from 1 to 8, alternatively from 2 to 8, alternatively from 3 to 8, or from 4 to 8; and $R^1$ moieties are independently selected from $C_1$-$C_{22}$ alkyl or $C_1$-$C_{30}$ alkoxy, linear or branched, cyclic or acyclic, saturated or unsaturated, substituted or unsubstituted.

In one embodiment, the $R^1$ moieties comprise linear alkyl or alkoxy moieties having independently selected and varying chain length. For example, $R^1$ may comprise a mixture of linear alkyl or alkoxy moieties wherein greater than about 20% of the linear chains are $C_{18}$, alternatively greater than about 50% of the linear chains are $C_{18}$, alternatively greater than about 80% of the linear chains are $C_{18}$.

In another embodiment, the $R^1$ moieties comprise a mixture of saturate and unsaturated alkyl or alkoxy moieties; the degree of unsaturation can be measured by "Iodine Value" (hereinafter referred as "IV", as measured by the standard AOCS method). The IV of the sucrose esters suitable for use herein ranges from about 1 to about 150, or from about 2 to about 100, or from about 5 to about 85. The $R^1$ moieties may be hydrogenated to reduce the degree of unsaturation. In the case where a higher IV is preferred, preferably from about 40 to about 95, then oleic acid and fatty acids derived from soybean oil and canola oil are the preferred starting materials.

In a further embodiment, the unsaturated $R^1$ moieties may comprise a mixture of "cis" and "trans" forms about the unsaturated sites. The "cis" / "trans" ratios may range from about 1:1 to about 50:1, or from about 2:1 to about 40:1, or from about 3:1 to about 30:1, or from about 4:1 to about 20:1.

Non-limiting examples of water insoluble fabric care benefit agents include dispersible polyethylene and polymer latexes. These agents can be in the form of emulsions, latexes, dispersions, suspensions, and the like. Preferably they are in the form of an emulsion or a latex. Dispersible polyethylenes and polymer latexes can have a wide range of particle size diameters ($\chi_{50}$ ) including but not limited to from about 1 nm to about 100 um; alternatively from about 10 nm to about 10 um. As such, the preferred particle sizes of dispersible polyethylenes and polymer latexes are generally, but without limitation, smaller than silicones or other fatty oils.

Generally, any surfactant suitable for making polymer emulsions or emulsion polymerizations of polymer latexes can be used to make the water insoluble fabric care benefit agents of the present invention. Suitable surfactants consist of emulsifiers for polymer emulsions and latexes, dispersing agents for polymer dispersions and suspension agents for polymer suspensions. Suitable surfactants include anionic, cationic, and nonionic surfactants, or combinations thereof. Nonionic and anionic surfactants are preferred. In one embodiment, the ratio of surfactant to polymer in the water insoluble fabric care benefit agent is about 1:100 to about 1:2; alternatively from about 1:50 to about 1:5, respectively. Suitable water insoluble fabric care benefit agents include but are not limited to the examples described below.

Dispersible Polyolefins

Generally, all dispersible polyolefins that provide fabric care benefits can be used as water insoluble fabric care benefit agents in the present invention. The polyolefins can be in the format of waxes, emulsions, dispersions or suspensions. Non-limiting examples are discussed below.

In one embodiment, the polyolefin is chosen from a polyethylene, polypropylene, or a combination thereof. The polyolefin may be at least partially modified to contain various functional groups, such as carboxyl, alkylamide, sulfonic acid or amide groups. In another embodiment, the polyolefin is at least partially carboxyl modified or, in other words, oxidized.

For ease of formulation, the dispersible polyolefin may be introduced as a suspension or an emulsion of polyolefin dispersed by use of an emulsifying agent. The polyolefin suspension or emulsion preferably comprises from about 1 % to about 60%, alternatively from about 10% to about 55%, alternatively from about 20% to about 50% by weight of polyolefin. The polyolefin preferably has a wax dropping point (see ASTM D3954- 94, volume 15.04 --- "Standard Test Method for Dropping Point of Waxes") from about 20° to about 170°C, alternatively from about 50° to about 140°C. Suitable polyethylene waxes are available commercially from suppliers including but not limited to Honeywell (A-C polyethylene), Clariant (Velustrol® emulsion), and BASF (LUWAX®).

When an emulsion is employed with the dispersible polyolefin, the emulsifier may be any suitable emulsification agent. Non-limiting examples include an anionic, cationic, nonionic surfactant, or a combination thereof. However, almost any suitable surfactant or suspending agent may be employed as the emulsification agent. The dispersible polyolefin is dispersed by use of an emulsification agent in a ratio to polyolefin wax of about 1:100 to about 1:2, alternatively from about 1:50 to about 1:5, respectively.

Polymer Latexes

Polymer latex is made by an emulsion polymerization which includes one or more monomers, one or more emulsifiers, an initiator, and other components familiar to those of ordinary skill in the art. Generally, all polymer latexes that provide fabric care benefits can be used as water insoluble fabric care benefit agents of the present invention. Non-limiting examples of suitable polymer latexes include those disclosed in WO 02/18451; US 2004/0038851 A1; and US 2004/0065208 A1. Additional non-limiting examples include the monomers used in producing polymer latexes such as: (1) 100% or pure butylacrylate; (2) butylacrylate and butadiene mixtures with at least 20% (weight monomer ratio) of butylacrylate; (3) butylacrylate and less than 20% (weight monomer ratio) of other monomers excluding butadiene; (4) alkylacrylate with an alkyl carbon chain at or greater than $C_6$; (5) alkylacrylate with an alkyl carbon chain at or greater than $C_6$ and less than 50% (weight monomer ratio) of other monomers; (6) a third monomer (less than 20% weight monomer ratio) added into an aforementioned monomer systems; and (7) combinations thereof.

Polymer latexes that are suitable fabric care benefit agents in the present invention may include those having a glass transition temperature of from about -120°C to about 120°C, alternatively from about -80°C to about 60°C. Suitable emulsifiers include anionic, cationic, nonionic and amphoteric surfactants. Suitable initiators include initiators that are suitable for emulsion polymerization of polymer latexes. The particle size diameter ($\chi_{50}$) of the polymer latexes can be from about 1 nm to about 10 $\mu$m, alternatively from about 10 nm to about 1 $\mu$m, preferably from about 10 nm to about 20 nm.

Fatty Acid

One aspect of the invention provides a fabric softening composition comprising a fatty acid, preferably a free fatty acid. The term "fatty acid" is used herein in the broadest sense to include unprotonated or protonated forms of a fatty acid; and includes fatty acid that is bound or unbound to another chemical moiety as well as the various combinations of these species of fatty acid. One skilled in the art will readily appreciate that the pH of an aqueous composition will dictate, in part, whether a fatty acid is protonated or unprotonated. In another embodiment, the fatty acid is in its unprotonated, or salt form, together with a counter ion, such as, but not limited to, calcium, magnesium, sodium, potassium and the like. The term "free fatty acid" means a fatty acid that is not bound (to another chemical moiety (covalently or otherwise) to another chemical moiety.

In one embodiment, the fatty acid may include those containing from about 12 to about 25, preferably from about 13 to about 22, more preferably from about 16 to about 20, total carbon atoms, with the fatty moiety containing from about 10 to about 22, preferably from about 12 to about 18, more preferably from about 14 (mid-cut) to about 18 carbon atoms.

The fatty acids of the present invention may be derived from (1) an animal fat, and/or a partially hydrogenated animal fat, such as beef tallow, lard, etc.; (2) a vegetable oil, and/or a partially hydrogenated vegetable oil such as canola oil, safflower oil, peanut oil, sunflower oil, sesame seed oil, rapeseed oil, cottonseed oil, corn oil, soybean oil, tall oil, rice bran oil, palm oil, palm kernel oil, coconut oil, other tropical palm oils, linseed oil, tung oil, etc. ; (3) processed and/or

bodied oils, such as linseed oil or tung oil via thermal, pressure, alkali-isomerization and catalytic treatments; (4) a mixture thereof, to yield saturated (e.g. stearic acid), unsaturated (e.g. oleic acid), polyunsaturated (linoleic acid), branched (e.g. isostearic acid) or cyclic (e.g. saturated or unsaturated $\alpha$-disubstituted cyclopentyl or cyclohexyl derivatives of polyunsaturated acids) fatty acids. Non-limiting examples of fatty acids (FA) are listed in U.S. Pat. No. 5,759,990 at col 4, lines 45-66.

Mixtures of fatty acids from different fat sources can be used, and in some embodiments preferred.

It is preferred that at least a majority of the fatty acid that is present in the fabric softening composition of the present invention is unsaturated, e.g., from about 40% to 100%, preferably from about 55% to about 99%, more preferably from about 60% to about 98%, by weight of the total weight of the fatty acid present in the composition, although fully saturated and partially saturated fatty acids can be used. As such, it is preferred that the total level of polyunsaturated fatty acids (TPU) of the total fatty acid of the inventive composition is preferably from about 0% to about 75% by weight of the total weight of the fatty acid present in the composition.

The cis/trans ratio for the unsaturated fatty acids may be important, with the cis/trans ratio (of the C18:1 material) being from at least about 1:1, preferably at least about 3:1, more preferably from about 4:1, and even more preferably from about 9:1 or higher.

Branched fatty acids such as isostearic acid are also preferred since they may be more stable with respect to oxidation and the resulting degradation of color and odor quality.

The Iodine Value or "IV" measures the degree of unsaturation in the fatty acid. In one embodiment of the invention, the fatty acid has an IV preferably from about 40 to about 140, more preferably from about 50 to about 120 and even more preferably from about 85 to about 105.

Softening Oils

Another class of optional fabric care actives is softening oils, which include but are not limited to, vegetable oils (such as soybean, sunflower, and canola), hydrocarbon based oils (natural and synthetic petroleum lubricants, preferably polyolefins, isoparaffins, and cyclic paraffins), triolein, fatty esters, fatty alcohols, fatty amines, fatty amides, and fatty ester amines. Oils can be combined with fatty acid softening agents, clays, and silicones.

Clays

In one embodiment of the invention, the fabric care composition may comprise a clay as a fabric care active. In one embodiment clay can be a softener or co-softeners with another softening active, for example, silicone. Preferred clays include those materials classified geologically smectites and are described in U.S. Pat. Appl. Publ. 20030216274 A1, to Valerio Del Duca, et al., published Nov. 20, 2003, paragraphs 107- 120.

Other suitable clays are described U.S. Pat. Nos. 3,862,058; 3,948,790; 3,954,632; 4,062,647; and U.S. Patent Application Publication No. 20050020476A1 to Wahl, et. al., page 5 and paragraph 0078 through page 6 and paragraph 0087.

Adjunct Materials

While not essential for the purposes of the present invention, the non-limiting list of adjuncts illustrated hereinafter are suitable for use in the instant compositions and may be desirably incorporated in certain embodiments of the invention, for example to assist or enhance performance, for treatment of the substrate to be cleaned, or to modify the aesthetics of the composition as is the case with perfumes, colorants, dyes or the like. It is understood that such adjuncts are in addition to the components that are supplied via Applicants' delivery particles and FSAs. The precise nature of these additional components, and levels of incorporation thereof, will depend on the physical form of the composition and the nature of the operation for which it is to be used. Suitable adjunct materials include, but are not limited to, surfactants, builders, chelating agents, dye transfer inhibiting agents, dispersants, enzymes, and enzyme stabilizers, catalytic materials, bleach activators, polymeric dispersing agents, clay soil removal/anti-redeposition agents, brighteners, suds suppressors, dyes, additional perfume and perfume delivery systems, structure elasticizing agents, fabric softeners, carriers, hydrotropes, processing aids and/or pigments. In addition to the disclosure below, suitable examples of such other adjuncts and levels of use are found in U.S. Patent Nos. 5,576,282, 6,306,812 B1 and 6,326,348 B1 that are incorporated by reference.

As stated, the adjunct ingredients are not essential to Applicants' cleaning and fabric care compositions. Thus, certain embodiments of Applicants' compositions do not contain one or more of the following adjuncts materials: bleach activators, surfactants, builders, chelating agents, dye transfer inhibiting agents, dispersants, enzymes, and enzyme stabilizers, catalytic metal complexes, polymeric dispersing agents, clay and soil removal/anti-redeposition agents, brighteners, suds suppressors, dyes, additional perfumes and perfume delivery systems, structure elasticizing agents,

fabric softeners, carriers, hydrotropes, processing aids and/or pigments. However, when one or more adjuncts is present, such one or more adjuncts may be present as detailed below:

Surfactants - The compositions according to the present invention can comprise a surfactant or surfactant system wherein the surfactant can be selected from nonionic and/or anionic and/or cationic surfactants and/or ampholytic and/or zwitterionic and/or semi-polar nonionic surfactants. The surfactant is typically present at a level of from about 0.1%, from about 1%, or even from about 5% by weight of the cleaning compositions to about 99.9%, to about 80%, to about 35%, or even to about 30% by weight of the cleaning compositions.

Builders - The compositions of the present invention can comprise one or more detergent builders or builder systems. When present, the compositions will typically comprise at least about 1% builder, or from about 5% or 10% to about 80%, 50%, or even 30% by weight, of said builder. Builders include, but are not limited to, the alkali metal, ammonium and alkanolammonium salts of polyphosphates, alkali metal silicates, alkaline earth and alkali metal carbonates, aluminosilicate builders polycarboxylate compounds. ether hydroxypolycarboxylates, copolymers of maleic anhydride with ethylene or vinyl methyl ether, 1,3,5-trihydroxybenzene-2,4,6-trisulphonic acid, and carboxymethyl-oxysuccinic acid, the various alkali metal, ammonium and substituted ammonium salts of polyacetic acids such as ethylenediamine tetraacetic acid and nitrilotriacetic acid, as well as polycarboxylates such as mellitic acid, succinic acid, oxydisuccinic acid, polymaleic acid, benzene 1,3,5-tricarboxylic acid, carboxymethyloxysuccinic acid, and soluble salts thereof.

Chelating Agents - The compositions herein may also optionally contain one or more copper, iron and/or manganese chelating agents. If utilized, chelating agents will generally comprise from about 0.1% by weight of the compositions herein to about 15%, or even from about 3.0% to about 15% by weight of the compositions herein.

Dye Transfer Inhibiting Agents - The compositions of the present invention may also include one or more dye transfer inhibiting agents. Suitable polymeric dye transfer inhibiting agents include, but are not limited to, polyvinylpyrrolidone polymers, polyamine N-oxide polymers, copolymers of N-vinylpyrrolidone and N-vinylimidazole, polyvinyloxazolidones and polyvinylimidazoles or mixtures thereof. When present in the compositions herein, the dye transfer inhibiting agents are present at levels from about 0.0001 %, from about 0.01 %, from about 0.05% by weight of the cleaning compositions to about 10%, about 2%, or even about 1% by weight of the cleaning compositions.

Dispersants - The compositions of the present invention can also contain dispersants. Suitable water-soluble organic materials are the homo- or co-polymeric acids or their salts, in which the polycarboxylic acid may comprise at least two carboxyl radicals separated from each other by not more than two carbon atoms.

Enzymes - The compositions can comprise one or more detergent enzymes which provide cleaning performance and/or fabric care benefits. Examples of suitable enzymes include, but are not limited to, hemicellulases, peroxidases, proteases, cellulases, xylanases, lipases, phospholipases, esterases, cutinases, pectinases, keratanases, reductases, oxidases, phenoloxidases, lipoxygenases, ligninases, pullulanases, tannases, pentosanases, malanases, β-glucanases, arabinosidases, hyaluronidase, chondroitinase, laccase, and amylases, or mixtures thereof. A typical combination is a cocktail of conventional applicable enzymes like protease, lipase, cutinase and/or cellulase in conjunction with amylase.

Enzyme Stabilizers - Enzymes for use in compositions, for example, detergents can be stabilized by various techniques. The enzymes employed herein can be stabilized by the presence of water-soluble sources of calcium and/or magnesium ions in the finished compositions that provide such ions to the enzymes.

Catalytic Metal Complexes - Applicants' compositions may include catalytic metal complexes. One type of metal-containing bleach catalyst is a catalyst system comprising a transition metal cation of defined bleach catalytic activity, such as copper, iron, titanium, ruthenium, tungsten, molybdenum, or manganese cations, an auxiliary metal cation having little or no bleach catalytic activity, such as zinc or aluminum cations, and a sequestrate having defined stability constants for the catalytic and auxiliary metal cations, particularly ethylenediaminetetraacetic acid, ethylenediaminetetra (methyl-enephosphonic acid) and water-soluble salts thereof. Such catalysts are disclosed in U.S. patent 4,430,243.

If desired, the compositions herein can be catalyzed by means of a manganese compound. Such compounds and levels of use are well known in the art and include, for example, the manganese-based catalysts disclosed in U.S. patent 5,576,282.

Cobalt bleach catalysts useful herein are known, and are described, for example, in U.S. patents 5,597,936 and 5,595,967. Such cobalt catalysts are readily prepared by known procedures, such as taught for example in U.S. patents 5,597,936, and 5,595,967.

Compositions herein may also suitably include a transition metal complex of a macropolycyclic rigid ligand - abreviated as "MRL". As a practical matter, and not by way of limitation, the compositions and cleaning processes herein can be adjusted to provide on the order of at least one part per hundred million of the benefit agent MRL species in the aqueous washing medium, and may provide from about 0.005 ppm to about 25 ppm, from about 0.05 ppm to about 10 ppm, or even from about 0.1 ppm to about 5 ppm, of the MRL in the wash liquor.

Preferred transition-metals in the instant transition-metal bleach catalyst include manganese, iron and chromium. Preferred MRL's herein are a special type of ultra-rigid ligand that is cross-bridged such as 5,12-diethyl-1,5,8,12-tetraazabicyclo[6.6.2]hexa-decane.

Suitable transition metal MRLs are readily prepared by known procedures, such as taught for example in WO 00/32601, and U.S. patent 6,225,464.

Processes of Making and Using Compositions

The compositions of the present invention can be formulated into any suitable form and prepared by any process chosen by the formulator, non-limiting examples of which are described in U.S. 5,879,584; U.S. 5,691,297; U.S. 5,574,005; U.S. 5,569,645; U.S. 5,565,422; U.S. 5,516,448; U.S. 5,489,392; U.S. 5,486,303 all of which are incorporated herein by reference.

Method of Use

Compositions containing the benefit agent delivery particle disclosed herein can be used to clean or treat a situs *inter alia* a surface or fabric. Typically at least a portion of the situs is contacted with an embodiment of Applicants' composition, in neat form or diluted in a liquor, for example, a wash liquor and then the situs may be optionally washed and/or rinsed. In one aspect, a situs is optionally washed and/or rinsed, contacted with a particle according to the present invention or composition comprising said particle and then optionally washed and/or rinsed. For purposes of the present invention, washing includes but is not limited to, scrubbing, and mechanical agitation. The fabric may comprise most any fabric capable of being laundered or treated in normal consumer use conditions. Liquors that may comprise the disclosed compositions may have a pH of from about 3 to about 11.5. Such compositions are typically employed at concentrations of from about 500 ppm to about 15,000 ppm in solution. When the wash solvent is water, the water temperature typically ranges from about 5 ˚C to about 90 ˚C and, when the situs comprises a fabric, the water to fabric ratio is typically from about 1:1 to about 30:1.

TEST METHODS

It is understood that the test methods that are disclosed in the Test Methods Section of the present application must be used to determine the respective values of the parameters of Applicants' invention as such invention is described and claimed herein.

**(1) Particle size distribution**

a.) Place 1 gram of particles in 1 liter of distilled deionized (DI) water.
b.) Permit the particles to remain in the DI water for 10 minutes and then recover the particles by filtration.
c.) Determine the particle size distribution of the particle sample by measuring the particle size of 50 individual particles using the experimental apparatus and method of Zhang, Z.; Sun, G; "Mechanical Properties of Melamine-Formaldehyde microcapsules," J. Microencapsulation, vol 18, no. 5, pages 593-602, 2001.
d.) Average the 50 independent particle diameter measurements to obtain an average particle diameter.
e.) Use the 50 independent measurements to calculate a standard deviation of particle size using the following equation:

$$\mu = \sqrt{\frac{\sum (d-s)^2}{n-1}}$$

where

$\mu$ is the standard deviation
s is the average particle diameter
d is the independent particle diameter
n is the total number of particles whose diameter is measured.

**(2) Benefit Agent Retention Ratio**

a.) Add 1 gram of particle to 99 grams of composition that the particle will be employed in.

b.) Age the particle containing composition of a.) above for 2 weeks at 40˚C in a sealed, glass jar.
c.) Recover the particles from b.) above by filtration.
d.) Treat the particles of c.) above with a solvent that will extract all the benefit agent from the particles.
e.) Inject the benefit agent containing solvent from d.) above into a Gas Chromatograph and integrate the peak areas to determine the total quantity of benefit agent extracted from the particle sample.
f.) This quantity is then divided by the quantity that would be present if nothing had leaked out of the microcapsule (e.g. the total quantity of core material that is dosed into the composition via the microcapsules). This value is then multiplied by the ratio of average particle diameter to average particle thickness to obtain a Benefit Agent Retention Ratio.

A detailed analytical procedure to measure the Benefit Agent Retention Ratio is:

ISTD Solution

1. Weigh out 25mg dodecane into a weigh boat.
2. Rinse the dodecane into a 1000mL volumetric flask using ethanol.
3. Add ethanol to volume mark.
4. Stir solution until mixed. This solution is stable for 2 months.

Calibration Standard

1. Weigh out 75mg of core material into a 100 mL volumetric flask.
2. Dilute to volume with ISTD solution to from above. This standard solution is stable for 2 months.
3. Mix well.
4. Analyze via GC/FID.

Basic Sample Prep

(Prepare samples in triplicate)

1. Weigh 1.000 gram sample of aged composition containing particles into a 100 mL tri-pour beaker. Record weight..
2. Add 4 drops (approximately 0.1 gram) 2-ethyl-1,3-Hexanediol into the tri-pour beaker.
3. Add 50 mL Deionized water to the beaker. Stir for 1 minute.
4. Using a 60cc syringe, filter through a Millipore Nitrocellulose Filter Membrane (1.2 micron, 25 mm diameter).
5. Rinse through the filter with 10 mL of Hexane
6. Carefully remove the filter membrane and transfer to a 20 mL scintillation vial (using tweezers).
7. Add 10mL ISTD solution (as prepared above) to the scintillation vial containing the filter.
8. Cap tightly, mix, and heat vial at 60˚C for 30min.
9. Cool to room temperature.
10. Remove 1mL and filter through a 0.45-micron PTFE syringe filter into GC vial. Several PTFE filters may be required to filter a 1mL sample aliquot.
11. Analyze via GC/FID.

GG/FID Analysis Method:

Column - 30m X 0.25mm id, 1-um DB-1 phase
GC - 6890 GC equipped with EPC control and constant flow capability
Method - 50˚C, 1min. hold, temperature ramp of 4˚C/min. to 300˚C, and hold for 10min.
Injector - 1uL splitless injection at 240˚C

GC/FID Analysis Method - Microbore Column Method:

Column - 20m X 0.1mm id, 0.1μm DB-5
GC- 6890 GC equipped with EPC control and constant flow capability (constant flow 0.4mL/min)
Method - 50˚C, no hold, temperature ramp of 16˚C/min to 275˚C, and hold for 3min.
Injector - 1μL split injection (80:1 split) at 250˚C

Calculations:

$$\% \ Total \ Perfume = \frac{A_{IS} \ x \ W_{per\text{-}std} \ x \ A_{per\text{-}sam}}{A_{per\text{-}std} \ x \ A_{is\text{-}sam} \ x \ W_{sam}} \ x \ 100\%$$

where

$A_{is}$ = Area of internal standard in the core material calibration standard;
$W_{per\text{-}std}$ = weight of core material in the calibration sample
$A_{per\text{-}sam}$ = Area of core material peaks in the composition containing particle sample;
$A_{per\text{-}std}$ = Area of core material peaks in the calibration sample.
$A_{is\text{-}sam}$ = Area of internal standard in composition containing particle sample;
$W_{sam}$ = Weight of the composition containing particle sample

$$\mathrm{Re}tention_Ratio = \left( \frac{Total_Perfume}{Perfume_Dosed_Into_\mathrm{Pr}oduct_Via_Microcapsules} \right) \left( \frac{\mu}{T} \right)$$

where

µs the average particle diameter, from Test Method 1
T is the average particle thickness as calculated from Test Method 3

**(3) <u>Fracture Strength</u>**

a.) Place 1 gram of particles in 1 liter of distilled deionized (DI) water.
b.) Permit the particles to remain in the DI water for 10 minutes and then recover the particles by filtration.
c.) Determine the average rupture force of the particles by averaging the rupture force of 50 individual particles. The rupture force of a particle is determined using the procedure given in Zhang, Z.; Sun, G; "Mechanical Properties of Melamine-Formaldehyde microcapsules," J. Microencapsulation, vol 18, no. 5, pages 593-602, 2001. Then calculate the average fracture pressure by dividing the average rupture force (in Newtons) by the average cross-sectional area (as determined by Test Method 1 above) of the spherical particle ($\pi r^2$, where r is the radius of the particle before compression).
d.) Calculate the average fracture strength by using the following equation:

$$\sigma_{fracture_stress} = \frac{P}{4(d/T)}$$

where

P is the average fracture pressure from a.) above
d is the average diameter of the particle (as determined by Test Method 1 above)
T is the average shell thickness of the particle shell as determined by the following equation:

$$T = \frac{r_{capsule}(1-c)\rho_{perfume}}{3[c\rho_{wall} + (1-c)\rho_{perfume}]}$$

where

$c$ is the average perfume content in the particle
$r$ is the average particle radius
$\rho_{wall}$ is the average density of the shell as determined by ASTM method B923-02, "Standard Test Method for Metal Powder Skeletal Density by Helium or Nitrogen Pycnometry", ASTM International.
$\rho_{perfume}$ is the average density of the perfume as determined by ASTM method D1480-93(1997) "Standard Test Method for Density and Relative Density (Specific Gravity) of Viscous Materials by Bingham Pycnometer", ASTM International.

**(4) ClogP**
The "calculated logP" (ClogP) is determined by the fragment approach of Hansch and Leo (cf., A. Leo, in Comprehensive Medicinal Chemistry, Vol. 4, C. Hansch, P.G. Sammens, J.B. taylor, and C.A. Ramsden, Eds. P. 295, Pergamon Press, 1990, incorporated herein by reference). ClogP values may be calculated by using the "CLOGP" program available from Daylight Chemical Information Systems Inc. of Irvine, California U.S.A..
**(5) Boiling Point**
Boiling point is measured by ASTM method D2887-04a, "Standard Test Method for Boiling Range Distribution of Petroleum Fractions by Gas Chromatography," ASTM International.
**(6) Delivery Index Calculation**
The Delivery Index for a particle is calculated using the following equation:

$$Delivery_Index = \frac{\left[\left(\frac{\mu}{\sigma}\right)_{Particle_Size}\left(\frac{f_0}{f}\right)_{Fracture_Stress}\left(\frac{L/L_0}{t/\mu}\right)\right]}{100}$$

Where

$\mu$ is the average particle diameter
$\sigma$ is the standard deviation of the average particle diameter
$f_0$ is the minimum in-use fracture strength required to break the microcapsule
$f$ is the measured Fracture Strength
$(L/L_0)/(t/\mu)$ is the Benefit Agent Retention Ratio
t is the shell thickness of the particle

EXAMPLES

While particular embodiments of the present invention have been illustrated and described, it would be obvious to those skilled in the art that various other changes and modifications can be made without departing from the spirit and scope of the invention. It is therefore intended to cover in the appended claims all such changes and modifications that are within the scope of this invention.

EXAMPLE 1: 80 wt% Core / 20 wt% Wall Urea Based Polyurea Capsule

2 grams of Urea (Sigma Aldrich of Milwaukee, WI) is dissolved in 20g deionized water. 1 gram of resorcinol (Sigma Aldrich of Milwaukee, WI) is added to the homogeneous urea solution. 20 g of 37wt% formaldehyde solution (Sigma Aldrich of Milwaukee, WI) is added to the solution, and the pH of the slurry is adjusted to 8.0 using 1M sodium hydroxide solution (Sigma Aldrich of Milwaukee, WI). The reactants are allowed to sit at 35˚C for 2 hours. In a separate beaker, 80 grams of fragrance oil is added slowly to the urea-formaldehyde solution. The mixture is agitated using a Janke & Kunkel Laboretechnik mixer using a pitched, 3-blade agitator to achieve a 50 micron mean oil droplet size distribution. The pH of the slurry is adjusted to 3.0 using 1M Hydrochloric Acid to initiate the condensation reaction. The solution is heated to 65˚C and allowed to react in a constant temperature water bath, while slowly agitating the contents

of the mixture. The contents are allowed to react for 4 hours at 65˚C.

The Fracture Strength Test Method Apparatus is used to determine the average particle diameter, standard deviation of particle diameter. The average particle diameter of the microcapsules is 53 microns, with a standard deviation of 11 microns.

The mean rupture force of the encapsulated particles is measured to be 3.14 milliNewtons, the mean deformation of the particle at fracture is measured to be 26%. The thickness of the wall is calculated to be 1.24 microns, and the fracture strength is calculated to be 1.24 psia.

EXAMPLE 2: 85% Core / 15wt% Wall Melamine based Polyurea capsule

A first mixture is prepared by combining 208 grams of water and 5 grams of alkyl acrylate-acrylic acid copolymer ( Polysciences, Inc. of Warrington, Pennsylvania, USA). This first mixture is adjusted to pH 5.0 using acetic acid.

178 grams of the capsule core material which comprise a fragrance oil is added to the first mixture at a temperature of 45˚C to form an emulsion. The ingredients to form the capsule wall material are prepared as follows: 9 grams of a corresponding capsule wall material copolymer pre-polymer (butylacrylate-acrylic acid copolymer) and 90 grams of water are combined and adjusted to pH 5.0. To this mixture is added 28 grams of a partially methylated methylol melamine resin solution ("Cymel 385", 80% solids, Cytec). This mixture is added to the above described fragrance oil-in-water emulsion with stirring at a temperature of 45 degrees Centigrade. High speed blending is used to achieve a volume-mean particle size of 1 micron, and a standard deviation of 0.4 microns. The temperature of the mixture is gradually raised to 65 degrees Centigrade, and is maintained at this temperature overnight with continuous stirring to initiate and complete encapsulation.

To form the acrylic acid-alkyl acrylate copolymer capsule wall, the alkyl group can be selected from ethyl, propyl, butyl, amyl, hexyl, cyclohexyl, 2-ethylhexyl, or other alkyl groups having from one to about sixteen carbons, preferably one to eight carbons.

The Fracture Strength Test Method Apparatus is used to determine the average particle diameter, standard deviation of particle diameter. The mean rupture force of the encapsulated particles is measured to be 0.10 milliNewtons, the mean deformation of the particle at fracture is measured to be 60%. The thickness of the wall is calculated to be 0.02 microns, and the fracture strength is calculated to be 109 psia.

EXAMPLE 3: 90% Core / 10wt% Wall Melamine based Polyurea capsule

A first mixture is prepared by combining 208 grams of water and 5 grams of alkyl acrylate-acrylic acid copolymer (Polysciences, Inc. of Warrington, Pennsylvania, USA). This first mixture is adjusted to pH 5.0 using acetic acid.

280 grams of the capsule core material which comprise a fragrance oil is added to the first mixture at a temperature of 45˚C to form an emulsion. The ingredients to form the capsule wall material are prepared as follows: 9 grams of a corresponding capsule wall material copolymer pre-polymer (butylacrylate-acrylic acid copolymer) and 90 grams of water are combined and adjusted to pH 5.0. To this mixture is added 28 grams of a partially methylated methylol melamine resin solution ("Cymel 385", 80% solids, Cytec). This mixture is added to the above described fragrance oil-in-water emulsion with stirring at a temperature of 45 degrees Centigrade. High speed blending is used to achieve a volume-mean particle size of 14 micron, and a standard deviation of 2.6 microns. The temperature of the mixture is gradually raised to 65 degrees Centigrade, and is maintained at this temperature overnight with continuous stirring to initiate and complete encapsulation.

To form the acrylic acid-alkyl acrylate copolymer capsule wall, the alkyl group can be selected from ethyl, propyl, butyl, amyl, hexyl, cyclohexyl, 2-ethylhexyl, or other alkyl groups having from one to about sixteen carbons, preferably one to eight carbons.

The Fracture Strength Test Method Apparatus is used to determine the average particle diameter, standard deviation of particle diameter. The mean rupture force of the encapsulated particles is measured to be 1.66 milliNewtons, the mean deformation of the particle at fracture is measured to be 73%. The thickness of the wall is calculated to be 0.16 microns, and the fracture strength is calculated to be 9.3 psia.

EXAMPLE 4: 95% Core / 5wt% Wall Melamine based Polyurea capsule

A first mixture is prepared by combining 208 grams of water and 5 grams of alkyl acrylate-acrylic acid copolymer (Polysciences, Inc. of Warrington, Pennsylvania, USA). This first mixture is adjusted to pH 5.0 using acetic acid.

594 grams of the capsule core material which comprise a fragrance oil is added to the first mixture at a temperature of 45˚C to form an emulsion. The ingredients to form the capsule wall material are prepared as follows: 9 grams of a corresponding capsule wall material copolymer pre-polymer (butylacrylate-acrylic acid copolymer) and 90 grams of water are combined and adjusted to pH 5.0. To this mixture is added 28 grams of a partially methylated methylol

melamine resin solution ("Cymel 385", 80% solids, Cytec). This mixture is added to the above described fragrance oil-in-water emulsion with stirring at a temperature of 45 degrees Centigrade. High speed blending is used to achieve a volume-mean particle size of 11 micron, and a standard deviation of 3.2 microns. The temperature of the mixture is gradually raised to 65 degrees Centigrade, and is maintained at this temperature overnight with continuous stirring to initiate and complete encapsulation.

To form the acrylic acid-alkyl acrylate copolymer capsule wall, the alkyl group can be selected from ethyl, propyl, butyl, amyl, hexyl, cyclohexyl, 2-ethylhexyl, or other alkyl groups having from one to about sixteen carbons, preferably one to eight carbons.

The Fracture Strength Test Method Apparatus is used to determine the average particle diameter, standard deviation of particle diameter. The mean rupture force of the encapsulated particles is measured to be 0.09 milliNewtons, the mean deformation of the particle at fracture is measured to be 25%. The thickness of the wall is calculated to be 0.062 microns, and the fracture strength is calculated to be 0.19 psia.

EXAMPLE 5: 80% Core / 20wt% Wall Melamine based Polyurea capsule

A first mixture is prepared by combining 208 grams of water and 5 grams of alkyl acrylate-acrylic acid copolymer (Polysciences, Inc. of Warrington, Pennsylvania, USA). This first mixture is adjusted to pH 5.0 using acetic acid.

125 grams of the capsule core material which comprises a fragrance oil is added to the first mixture at a temperature of 45˚C to form an emulsion. The ingredients to form the capsule wall material are prepared as follows: 9 grams of a corresponding capsule wall material copolymer pre-polymer (butylacrylate-acrylic acid copolymer) and 90 grams of water are combined and adjusted to pH 5.0. To this mixture is added 28 grams of a partially methylated methylol melamine resin solution ("Cymel 385", 80% solids, Cytec). This mixture is added to the above described fragrance oil-in-water emulsion with stirring at a temperature of 45 degrees Centigrade. High speed blending is used to achieve a volume-mean particle size of 8.7 micron, and a standard deviation of 3.3 microns. The temperature of the mixture is gradually raised to 65 degrees Centigrade, and is maintained at this temperature overnight with continuous stirring to initiate and complete encapsulation.

To form the acrylic acid-alkyl acrylate copolymer capsule wall, the alkyl group can be selected from ethyl, propyl, butyl, amyl, hexyl, cyclohexyl, 2-ethylhexyl, or other alkyl groups having from one to about sixteen carbons, preferably one to eight carbons.

The Fracture Strength Test Method Apparatus is used to determine the average particle diameter, standard deviation of particle diameter. The mean rupture force of the encapsulated particles is measured to be 1.10 milliNewtons, the mean deformation of the particle at fracture is measured to be 73%. The thickness of the wall is calculated to be 0.21 microns, and the fracture strength is calculated to be 15.8 psia.

EXAMPLE 6: 85% Core / 15wt% Wall Melamine based Polyurea capsule

A first mixture is prepared by combining 208 grams of water and 5 grams of alkyl acrylate-acrylic acid copolymer ( Polysciences, Inc. of Warrington, Pennsylvania, USA). This first mixture is adjusted to pH 5.0 using sodium hydroxide.

178 grams of the capsule core material which comprise a fragrance oil is added to the first mixture at a temperature of 65˚C to form an emulsion. High speed blending is used to achieve a volume-mean particle size of 1 micron. The ingredients to form the capsule wall material are prepared as follows: 9 grams of a corresponding capsule wall material copolymer pre-polymer (butylacrylate-acrylic acid copolymer) and 90 grams of water are combined and adjusted to pH 5.0. To this mixture is added 28 grams of a partially methylated methylol melamine resin solution ("Cymel 385", 80% solids, Cytec). This mixture is added to the above described fragrance oil-in-water emulsion with stirring at a temperature of 65 degrees Centigrade. The temperature of the mixture is maintained at this temperature for 8 hours with continuous stirring to initiate and complete encapsulation.

To form the acrylic acid-alkyl acrylate copolymer capsule wall, the alkyl group can be selected from ethyl, propyl, butyl, amyl, hexyl, cyclohexyl, 2-ethylhexyl, or other alkyl groups having from one to about sixteen carbons, preferably one to eight carbons.

The Fracture Strength Test Method Apparatus is used to determine the average particle diameter, standard deviation of particle diameter. The mean rupture force of the encapsulated particles is measured to be 0.10 milliNewtons, the mean deformation of the particle at fracture is measured to be 60%. The thickness of the wall is calculated to be 0.02 microns, and the fracture strength is calculated to be 109 psia.

EXAMPLE 7: 90% Core / 10wt% Wall Melamine based Polyurea capsule

A first mixture is prepared by combining 208 grams of water and 5 grams of alkyl acrylate-acrylic acid copolymer (Polysciences, Inc. of Warrington, Pennsylvania, USA). This first mixture is adjusted to pH 5.0 using sodium hydroxide.

280 grams of the capsule core material which comprise a fragrance oil is added to the first mixture at a temperature of 65˚C to form an emulsion. High speed blending is used to achieve a volume-mean particle size of 14 microns. The ingredients to form the capsule wall material are prepared as follows: 9 grams of a corresponding capsule wall material copolymer pre-polymer (butylacrylate-acrylic acid copolymer) and 90 grams of water are combined and adjusted to pH 5.0. To this mixture is added 28 grams of a partially methylated methylol melamine resin solution ("Cymel 385", 80% solids, Cytec). This mixture is added to the above described fragrance oil-in-water emulsion with stirring at a temperature of 65 degrees Centigrade. The temperature of the mixture is maintained at this temperature for 8 hours with continuous stirring to initiate and complete encapsulation.

To form the acrylic acid-alkyl acrylate copolymer capsule wall, the alkyl group can be selected from ethyl, propyl, butyl, amyl, hexyl, cyclohexyl, 2-ethylhexyl, or other alkyl groups having from one to about sixteen carbons, preferably one to eight carbons.

The Fracture Strength Test Method Apparatus is used to determine the average particle diameter, standard deviation of particle diameter. The mean rupture force of the encapsulated particles is measured to be 1.66 milliNewtons, the mean deformation of the particle at fracture is measured to be 73%. The thickness of the wall is calculated to be 0.16 microns, and the fracture strength is calculated to be 9.3 psia.

EXAMPLE 8: 95% Core / 5wt% Wall Melamine based Polyurea capsule

A first mixture is prepared by combining 208 grams of water and 5 grams of alkyl acrylate-acrylic acid copolymer (Polysciences, Inc. of Warrington, Pennsylvania, USA). This first mixture is adjusted to pH 5.0 using sodium hydroxide.

594 grams of the capsule core material which comprise a fragrance oil is added to the first mixture at a temperature of 65˚C to form an emulsion. High speed blending is used to achieve a volume-mean particle size of 11 microns. The ingredients to form the capsule wall material are prepared as follows: 9 grams of a corresponding capsule wall material copolymer pre-polymer (butylacrylate-acrylic acid copolymer) and 90 grams of water are combined and adjusted to pH 5.0. To this mixture is added 28 grams of a partially methylated methylol melamine resin solution ("Cymel 385", 80% solids, Cytec). This mixture is added to the above described fragrance oil-in-water emulsion with stirring at a temperature of 65 degrees Centigrade. The temperature of the mixture is maintained at this temperature for 8 hours with continuous stirring to initiate and complete encapsulation.

To form the acrylic acid-alkyl acrylate copolymer capsule wall, the alkyl group can be selected from ethyl, propyl, butyl, amyl, hexyl, cyclohexyl, 2-ethylhexyl, or other alkyl groups having from one to about sixteen carbons, preferably one to eight carbons.

The Fracture Strength Test Method Apparatus is used to determine the average particle diameter, standard deviation of particle diameter. The mean rupture force of the encapsulated particles is measured to be 0.09 milliNewtons, the mean deformation of the particle at fracture is measured to be 25%. The thickness of the wall is calculated to be 0.062 microns, and the fracture strength is calculated to be 0.19 psia.

EXAMPLE 9: 80% Core / 20wt% Wall Melamine based Polyurea capsule

A first mixture is prepared by combining 208 grams of water and 5 grams of alkyl acrylate-acrylic acid copolymer (Polysciences, Inc. of Warrington, Pennsylvania, USA). This first mixture is adjusted to pH 5.0 using sodium hydroxide.

125 grams of the capsule core material which comprises a fragrance oil is added to the first mixture at a temperature of 65˚C to form an emulsion. High speed blending is used to achieve a volume-mean particle size of 8.7 microns. The ingredients to form the capsule wall material are prepared as follows: 9 grams of a corresponding capsule wall material copolymer pre-polymer (butylacrylate-acrylic acid copolymer) and 90 grams of water are combined and adjusted to pH 5.0. To this mixture is added 28 grams of a partially methylated methylol melamine resin solution ("Cymel 385", 80% solids, Cytec). This mixture is added to the above described fragrance oil-in-water emulsion with stirring at a temperature of 65 degrees Centigrade. The temperature of the mixture is maintained at this temperature for 8 hours with continuous stirring to initiate and complete encapsulation.

To form the acrylic acid-alkyl acrylate copolymer capsule wall, the alkyl group can be selected from ethyl, propyl, butyl, amyl, hexyl, cyclohexyl, 2-ethylhexyl, or other alkyl groups having from one to about sixteen carbons, preferably one to eight carbons.

EXAMPLE 10

Using the microcapsule formation process of Example 2 or 6, the average particle diameter is 3.6 microns, and standard deviation of 1.2 microns. The mean rupture force of the encapsulated particles is measured to be 0.61 milliNewtons, the mean deformation of the particle at fracture is measured to be 77%. The thickness of the wall is calculated to be 0.06 microns, and the fracture strength is calculated to be 51.3 psia.

EXAMPLE 11

Using the microcapsule formation process of Example 2 or 6, the average particle diameter is 9.5 microns, and standard deviation of 3.0 microns. The mean rupture force of the encapsulated particles is measured to be 0.46 milliNewtons, the mean deformation of the particle at fracture is measured to be 61 %. The thickness of the wall is calculated to be 0.16 microns, and the fracture strength is calculated to be 5.6 psia.

EXAMPLE 12

Using the microcapsule formation process of Example 3 or 7, the average particle diameter is 17 microns, and standard deviation of 3.3 microns. The mean rupture force of the encapsulated particles is measured to be 1.54 milliNewtons, the mean deformation of the particle at fracture is measured to be 68%. The thickness of the wall is calculated to be 0.19 microns, and the fracture strength is calculated to be 5.9 psia

EXAMPLE 13

Using the microcapsule formation process of Example 3 or 7, the average particle diameter is 26 microns, and standard deviation of 9.3 microns. The mean rupture force of the encapsulated particles is measured to be 3.45 milliNewtons, the mean deformation of the particle at fracture is measured to be 71%. The thickness of the wall is calculated to be 0.30 microns, and the fracture strength is calculated to be 5.5 psia

EXAMPLE 14

Using the microcapsule formation process of Example 2 or 6, the average particle diameter is 6.8 microns, and standard deviation of 2.6 microns. The mean rupture force of the encapsulated particles is measured to be 0.26 milliNewtons, the mean deformation of the particle at fracture is measured to be 68%. The thickness of the wall is calculated to be 0.12 microns, and the fracture strength is calculated to be 4.5 psia

EXAMPLE 15

Using the microcapsule formation process of Example 1, wherein average particle diameter is 62 microns, and standard deviation of 12 microns. The mean rupture force of the encapsulated particles is measured to be 3.45 milliNewtons, the mean deformation of the particle at fracture is measured to be 23%. The thickness of the wall is calculated to be 1.46 microns, and the fracture strength is calculated to be 1.0 psia

EXAMPLE 16 Leakage of Fragrance Oil From Particles

The particles described in Examples 1 through 15 are incorporated into the following Fabric Softener composition via simple blending, to deliver 0.60 wt% of the fragrance oil into the formula via the microcapsules. The following are non-limiting examples of the fabric care compositions of the present invention.

| | EXAMPLE 12 FORMULATIONS | | | | | | | | | |
|---|---|---|---|---|---|---|---|---|---|---|
| (%wt) | I | II | III | IV | V | VI | VII | VIII | IX | X |
| FSA [a] | 14-16.5 | 14-16.5 | 14-16.5 | 14-16.5 | 14-16.5 | 14-16.5 | 14-16.5 | 14-16.5 | 14-16.5 | 14-16.5 |
| Ethanol | 2.2-2.6 | 2.2-2.6 | 2.2-2.6 | 2.2-2.6 | 2.2-2.6 | 2.2-2.6 | 2.2-2.6 | 2.2-2.6 | 2.2-2.6 | 2.2-2.6 |
| Starch [b] | 1.25-1.5 | 1.25-1.5 | 1.25-1.5 | 1.25-1.5 | 1.25-1.5 | 1.25-1.5 | 1.25-1.5 | 1.25-1.5 | 1.25-1.5 | 1.25-1.5 |
| Perfume | 0.8-1.5 | 0.8-1.5 | 0.8-1.5 | 0.8-1.5 | 0.8-1.5 | 0.8-1.5 | 0.8-1.5 | 0.8-1.5 | 0.8-1.5 | 0.8-1.5 |
| Encapsulated Perfume | 0.6 | 0.6 | 0.6 | 0.6 | 0.6 | 0.60 | 0.6 | 0.6 | 0.6 | 0.6 |
| Formaldehyde Scavenger[i] | 0.03 | 0.03 | 0.03 | 0.03 | 0.03 | 0.03 | 0.03 | 0.03 | 0.03 | 0.03 |
| Phase Stabilizing Polymer[c] | 0.14-0.21 | 0.14-0.21 | 0.14-0.21 | 0.14-0.21 | 0.14-0.21 | 0.14-0.21 | 0.14-0.21 | 0.14-0.21 | 0.14-0.21 | 0.14-0.21 |
| Calcium Chloride | 0.1-0.3 | 0.1-0.3 | 0.1-0.3 | 0.1-0.3 | 0.1-0.3 | 0.1-0.3 | 0.1-0.3 | 0.1-0.3 | 0.1-0.3 | 0.1-0.3 |
| DTPA[d] | 0.017 | 0.017 | 0.017 | 0.017 | 0.017 | 0.017 | 0.017 | 0.017 | 0.017 | 0.017 |
| Preservative (ppm)[e] | 5 | 5 | 5 | 5 | 5 | 5 | 5 | 5 | 5 | 5 |
| Antifoam[f] | 0.015 | 0.018 | 0.015 | 0.015 | 0.015 | 0.015 | 0.015 | 0.015 | 0.015 | 0.015 |
| Dye (ppm) | 30-300 | 30-300 | 30-300 | 30-300 | 30-300 | 30-300 | 30-300 | 30-300 | 30-300 | 30-300 |
| Ammonium Chloride | 0.02-0.12 | 0.02-0.12 | 0.02-0.12 | 0.02-0.12 | 0.02-0.12 | 0.02-0.12 | 0.02-0.12 | 0.02-0.12 | 0.02-0.12 | 0.02-0.12 |
| HC1 | 0.012 | 0.014 | 0.012 | 0.012 | 0.028 | 0.028 | 0.016 | 0.025 | 0.011 | 0.011 |
| Structurant[g] | 0.01 | 0.01 | 0.01 | 0.01 | 0.01 | 0.01 | 0.01 | 0.01 | 0.01 | 0.01 |

(continued)

| | EXAMPLE 12 FORMULATIONS | | | | | | | | | |
|---|---|---|---|---|---|---|---|---|---|---|
| (%wt) | I | II | III | IV | V | VI | VII | VIII | IX | X |
| Deionized Water | Balance | Balance | Balance | Balance | Balance | Balance | Balance | Balance | Balance | Balance |
| Microcapsule Example # | 10 | 5 | 4 | 2 | 6 | 7 | 3 | 8 | 9 | 1 |
| Perfume In Capsule[h] | 86% | 80% | 62% | 5% | 20% | 86% | 74% | 74% | 80% | 1% |

[a] N,N-di(tallowoyloxyethyl)-N,N-dimethylammonium chloride.

[b] Cationic high amylose maize starch available from National Starch under the trade name CATO®.

[c] Copolymer of ethylene oxide and terephthalate having the formula described in US 5,574,179 at col.15, lines 1-5, wherein each X is methyl, each n is 40, u is 4, each R1 is essentially 1,4-phenylene moieties, each R2 is essentially ethylene, 1,2-propylene moieties, or mixtures thereof.

[d] Diethylenetriaminepentaacetic acid.

[e] KATHON® CG available from Rohm and Haas Co. "PPM" is "parts per million."

[f] Silicone antifoam agent available from Dow Coming Corp. under the trade name DC2310.

[g] Hydrophobically-modified ethoxylated urethane available from Rohm and Haas under the tradename Aculan 44.

[h] Quantity of perfume that has leaked out of microcapsule particle after ageing the composition containing the particle for 2 weeks at 40C, reflected as % of original perfume remaining in the particle.

[i] The formaldehyde scavenger is acetoacetamide available from Aldrich.

Next, the Delivery Index for each particle is calculated according to Test Method 6. Where $f_0$ is 109 psia, and $f_0$ is determined by depositing microcapsules of various fracture strengths onto cotton terry fabric. Next, a perfume expert rubs the fabric and determines the intensity and character of odor delivered in the headspace of the fabric. The $f_0$ is the minimum fracture strength at which the perfumer notices a consumer-noticeable odor intensity increase upon rubbing the fabric.

| Example | Particle Diameter (microns) | Std. Dev. In Particle Diameter (microns) | Fracture Strength (psia) | Perfume Retention Ratio % | Wall Thickness (micron) | Delivery Index | Dry Fabric Odor Scale Pre-Rub / Post-Rub |
|---|---|---|---|---|---|---|---|
| No capsules | N/A | N/A | N/A | N/A | N/A | N/A | 35/35 |
| 10 | 6.8 | 2.6 | 4.51 | 86% | 0.1182 | 31.2 | 45/60 |
| 5 | 8.7 | 3.3 | 15.84 | 80% | 0.2053 | 6.2 | 45/55 |
| 4 | 11.1 | 3.2 | 0.19 | 62% | 0.0621 | 2221.0 | 40/55 |
| 2 | 1 | 0.4 | 108.97 | 5% | 0.02 | 0.072 | 30/35 |
| 6 | 3.6 | 1.2 | 51.29 | 20% | 0.06 | 0.733 | 30/45 |
| 7 | 9.46 | 2.93 | 5.60 | 86% | 0.16 | 31.081 | 45/60 |
| 3 | 13.92 | 2.64 | 9.31 | 74% | 0.16 | 40.103 | 45/55 |
| 8 | 16.92 | 3.33 | 5.88 | 74% | 0.19 | 61.260 | 45/55 |
| 9 | 26.1 | 9.3 | 5.52 | 80% | 0.30 | 38.946 | 35/45 |
| 1 | 52.55 | 10.85 | 1.24 | 1% | 1.24 | 1.802 | 30/35 |
| 11 | 61.9 | 12.2 | 1.05 | 1% | 1.46 | 2.227 | 30/35 |

A difference of 7 points on the Dry Fabric Odor scale is consumer noticeable.

EXAMPLE 17

Non-limiting examples of product formulations containing microcapsules are summarized in the following table.

| | EXAMPLES | | | | | | | | | |
|---|---|---|---|---|---|---|---|---|---|---|
| (%wt) | XI | XII | XIII | XIV | XV | XVI | XVII | XVIII | XIX | XX |
| FSA [a] | 14 | 16.47 | 14 | 12 | 12 | 16.47 | --- | --- | 5 | 5 |
| FSA [b] | | | | | --- | | 3.00 | --- | --- | --- |
| FSA [c] | | | | | --- | | --- | 6.5 | --- | --- |
| Ethanol | 2.18 | 2.57 | 2.18 | 1.95 | 1.95 | 2.57 | --- | --- | 0.81 | 0.81 |
| Isopropyl Alcohol | --- | --- | --- | --- | --- | --- | 0.33 | 1.22 | --- | --- |
| Starch[d] | 1.25 | 1.47 | 2.00 | 1.25 | --- | 2.30 | 0.5 | 0.70 | 0.71 | 0.42 |
| Microcapsule (% active) | 0.6 | 0.75 | 0.6 | 0.75 | 0.37 | 0.60 | 0.37 | 0.6 | 0.37 | 0.37 |
| Formaldehyde Scavenger[e] | 0.40 | 0.13 | 0.065 | 0.25 | 0.03 | 0.030 | 0.030 | 0.065 | 0.03 | 0.03 |
| Phase Stabilizing Polymer[f] | 0.21 | 0.25 | 0.21 | 0.21 | 0.14 | --- | --- | 0.14 | --- | --- |
| Suds Suppressor[g] | --- | --- | --- | --- | --- | --- | --- | 0.1 | --- | --- |
| Calcium Chloride | 0.15 | 0.176 | 0.15 | 0.15 | 0.30 | 0.176 | --- | 0.1- | --- | --- |
| DTPA[h] | 0.017 | 0.017 | 0.017 | 0.017 | 0.007 | 0.007 | 0.20 | --- | 0.002 | 0.002 |
| Preservative (ppm) [i,j] | 5 | 5 | 5 | 5 | 5 | 5 | --- | 250[j] | 5 | 5 |
| Antifoam[k] | 0.015 | 0.018 | 0.015 | 0.015 | 0.015 | 0.015 | --- | --- | 0.015 | 0.015 |
| Dye (ppm) | 40 | 40 | 40 | 40 | 40 | 40 | 11 | 30-300 | 30 | 30 |
| Ammonium Chloride | 0.100 | 0.118 | 0.100 | 0.100 | 0.115 | 0.115 | --- | --- | --- | --- |
| HCl | 0.012 | 0.014 | 0.012 | 0.012 | 0.028 | 0.028 | 0.016 | 0.025 | 0.011 | 0.011 |
| Structurant[1] | 0.01 | 0.01 | 0.01 | 0.01 | 0.01 | 0.01 | 0.01 | 0.01 | 0.01 | 0.01 |
| Neat Unencapsulated Perfume | 0.8 | 0.7 | 0.9 | 0.5 | 1.2 | 0.5 | 1.1 | 0.6 | 1.0 | 0.9 |

(continued)

| | EXAMPLES | | | | | | | | | |
|---|---|---|---|---|---|---|---|---|---|---|
| (%wt) | XI | XII | XIII | XIV | XV | XVI | XVII | XVIII | XIX | XX |
| Deionized Water | Balance | Balance | Balance | Balance | Balance | Balance | Balance | Balance | Balance | Balance |

[a] N,N-di(tallowoyloxyethyl)-N,N-dimethylammonium chloride.
[b] Methyl bis(tallow amidoethyl)2-hydroxyethyl ammonium methyl sulfate.
[c] Reaction product of Fatty acid with Methyldiethanolamine in a molar ratio 1.5:1, quaternized with Methylchloride, resulting in a 1:1 molar mixture of N,N-bis(stearoyl-oxy-ethyl) N,N-dimethyl ammonium chloride and N-(stearoyl-oxy-ethyl) N,-hydroxyethyl N,N dimethyl ammonium chloride.
[d] Cationic high amylose maize starch available from National Starch under the trade name CATO®.
[e] The formaldehyde scavenger is acetoacetamide available from Aldrich.
[f] Copolymer of ethylene oxide and terephthalate having the formula described in US 5,574,179 at col.15, lines 1-5, wherein each X is methyl, each n is 40, u is 4, each R1 is essentially 1,4-phenylene moieties, each R2 is essentially ethylene, 1,2-propylene moieties, or mixtures thereof.
[g] SE39 from Wacker
[h] Diethylenetriaminepentaacetic acid.
[i] KATHON® CG available from Rohm and Haas Co. "PPM" is "parts per million."
[j] Gluteraldehyde
[k] Silicone antifoam agent available from Dow Coming Corp. under the trade name DC2310.
[l] Hydrophobically-modified ethoxylated urethane available from Rohm and Haas under the tradename Aculan 44.

EXAMPLE 18 Addition of Magnesium Chloride to a Microcapsule Dispersion

To 100 grams of the microcapsule dispersion of Example 2 is added 14.6 grams of a 33 wt% Magnesium Chloride solution (Chemical Ventures of Cincinnati, OH). Next, 10 grams of a 1 wt% Xanthan Gum solution (CP Kelco of San Jose, California) is added to the mixture. Then 3.0 grams of this mixture is then added to a 97 grams of fabric softener composition of Example 17, using a Janke Kunkel Laboretechnic mixer with a turbine, 3-blade agitator at 300-500 RPM for 2 minutes. There are no aggregates observed in the fabric softening composition.

EXAMPLE 19 Applying a coating of sodium silicate onto a microcapsule

To 171 grams of a dispersion of microcapsules containing 47wt% microcapsule particles of Example 2 is added 45 grams of sodium silicate 3.2R solution (44 wt% active, obtained from Akzo Nobel of Felling, U.K.) 154 grams of Deionized water is added to the slurry, and then pumped through a peristaltic pump into a centrifugal wheel nozzle rotating at 25,000 RPM, and situated in a co-current spray drying chamber (Niro, 3ft diameter). The atomized aqueous dispersion of microcapsules is spray dried at the following operating conditions: an inlet air temperature of 200˚C, an outlet air temperature of 95˚C, pressure drop of air is 42 millimeters of water (corresponds to 78 kg/hr airflow), the spray dryer is operated under a net negative pressure of -150 millimeters of water, and the pressure of air fed to the centrifugal atomizer is 5.0 barg. The dry particles are recovered from the collection vessel at the bottom of the spray dryer as well as from the cyclone, and mixed to form a homogeneous powder sample. The particles are found to have an average particle diameter of 50 micrometers. When the powder is added to a fabric care composition of Example 16 and aged for 4 weeks at 40C, less than 10% perfume loss is observed from the microcapsule particles.

EXAMPLE 20 Addition of Calcium Formate to Perfume Microcapsule Slurry

To 200 grams of a dispersion of microcapsules containing 47wt% microcapsule particles of Example 2 is added 30 grams of a 10wt% solution of calcium formate at a rate of 10 grams per minute. The slurry is then milled through a rotor stator mixer. The slurry is pumped through an Ultra Turrax T-25 mixer with a 25 mm diameter rotor-stator head with 1 mm diameter gap in the stator, at a rate of 160 grams per minute, and the rotor stator operating at 13,500 RPM (power drawn by the mixer per unit volume of 8 kW-hr/$m^3$).

EXAMPLE 21 Microcapsule Formation

Into 153 grams of a mixture of 149.5 grams of water and 3.5 grams of the acrylic acid-alkyl acrylate copolymer, adjusted to pH 5.0, are emulsified 180 grams of the intended capsule nucleus material solution of Table 2. A second mixture of 6.5 grams of the corresponding acrylic acid-alkyl acrylate copolymer and 65 grams of water is prepared and adjusted to pH 5.0 and 20 grams of a partially methylated methylol melamine resin solution ("Resimene 714", 80 percent solids, Monsanto Company, St. Louis, Mo.) is added and this mixture is in turn added with stirring to the above-described emulsion. The resulting mixture is placed in a container which is mounted in a room temperature water bath. Continuous stirring is provided and the bath is heated to 55 degrees C and maintained at this temperature, with continuous stirring, overnight to initiate and complete encapsulation. The resulting capsules are employed in any of the compositions of the present specification.

## Claims

1. A composition comprising a particle comprising a core material and a wall material that surrounds the core material, said particle having a Delivery Index of at least about 0.05 said composition being a consumer product.

2. The composition of Claim 1, wherein said particle's core material comprises a material selected from the group consisting of perfume, silicone oils, waxes, hydrocarbons, higher fatty acids, essential oils, lipids, skin coolants, vitamins, sunscreens, antioxidants, glycerine, catalysts, bleach particles, silicon dioxide particles, malodor reducing agents, dyes, brighteners, antibacterial actives, antiperspirant actives, cationic polymers and mixtures thereof.

3. The composition of Claim 1 wherein said particle's wall material comprises a material selected from the group consisting of polyamine, polyurea, polyurethane polysaccharides and modified polysaccharides, gel forming proteins, modified celluloses, carboxylic acid containing acrylic polymers, gelatin, gum arabic, urea crosslinked with formaldehyde, urea crosslinked with gluteraldehyde, melamine crosslinked with formaldehyde, chitin and chitosan and modified chitin and modified chitosan, sodium alginate, latexes, silicon dioxide, sodium silicates and mixtures

thereof.

**4.** The composition of Claim 1 wherein said particle comprises at least 1 weight % of a benefit agent.

**5.** The composition of Claim 4 wherein said particle comprises from about 20 to about 95 weight% of a benefit agent.

**6.** The composition of Claim 5 wherein said particle comprises from about 50 to about 90 weight% of a benefit agent.

**7.** The composition of Claim 6 wherein said particle comprises from about 80 to about 85 weight % of a benefit agent.

**8.** The composition of Claim 1 wherein said particle's core material comprises, based on total core material weight, at least about 20 wt% benefit agent.

**9.** The composition of Claim 4 wherein said benefit agent comprises a perfume composition, said particle comprising, based on total particle weight, from about 20 weight % to about 95 weight % of said perfume composition.

**10.** The composition of Claim 1 said composition comprising, based on total composition weight, from about 0.2 to about 10 weight % of said particle.

**11.** The composition of Claim 1, comprising a material selected from the group consisting of calcium formate, formic acid, polyamines and mixtures thereof.

**12.** A method of treating and/or cleaning a situs, said method comprising

a.) optionally washing and/or rinsing said situs;
b.) contacting said situs with a composition according to Claim 1; and
c.) optionally washing and/or rinsing said situs.

**13.** A situs treated with a composition according to Claim 1.

**14.** A method of improving the stability of a microcapsule slurry, comprising combining said microcapsule slurry with a material selected from the group consisting of calcium formate, formic acid and mixtures thereof, said material comprising, based on total slurry weight from about 0.6 wt. % to about 3 wt.% of said microcapsule slurry.

**15.** A composition according to Claims 1, said composition comprising a formaldehyde scavenger.

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- US 83458206 P
- US 77762906 P
- US 6869923 B1
- US P6592990 B2
- US P6544926 B1
- US 6592990 B
- US 4515705 A
- US 4537706 A
- US 4537707 A
- US 4550862 A
- US 4561998 A
- US 4597898 A
- US 4968451 A
- US 5565145 A
- US 5929022 A
- US 6294514 A
- US 6376445 A
- US P5759990 A
- US 20040204337 A1, Corona
- US 20040229769 A1, Smith
- US 6494920 B
- US 20020077265 A1, Buzzacarini
- WO 06007911 A1
- WO 06007899 A1
- US 20040142841 A1, Buzzaccarini
- WO 0218451 A
- US 20040038851 A1
- US 20040065208 A1
- US 5759990 A
- US 20030216274 A1, Valerio Del Duca
- US 3862058 A
- US 3948790 A
- US 3954632 A
- US 4062647 A
- US 20050020476 A1, Wahl
- US 5576282 A
- US 6306812 B1
- US 6326348 B1
- US 4430243 A
- US 5597936 A
- US 5595967 A
- WO 0032601 A
- US 6225464 B
- US 5879584 A
- US 5691297 A
- US 5574005 A
- US 5569645 A
- US 5565422 A
- US 5516448 A
- US 5489392 A
- US 5486303 A
- US 5574179 A

### Non-patent literature cited in the description


- **Zhang, Z. ; Sun, G.** Mechanical Properties of Melamine-Formaldehyde microcapsules. *J. Microencapsulation,* 2001, vol. 18 (5), 593-602
- **A. Leo.** Comprehensive Medicinal Chemistry. Pergamon Press, 1990, vol. 4, 295